(19) Europäisches Patentamt
European Patent Office
Office européen des brevets

(11) **EP 2 166 018 A2**

(12) **EUROPEAN PATENT APPLICATION**

(43) Date of publication:
**24.03.2010 Bulletin 2010/12**

(21) Application number: **09173597.7**

(22) Date of filing: **23.12.2005**

(51) Int Cl.:
***C07K 5/10*** *(2006.01)* ***C07K 5/08*** *(2006.01)*
***G01N 33/543*** *(2006.01)*

(84) Designated Contracting States:
**AT BE BG CH CY CZ DE DK EE ES FI FR GB GR HU IE IS IT LI LT LU LV MC NL PL PT RO SE SI SK TR**

(30) Priority: **23.12.2004 DK 200402010**
**13.01.2005 US 643314 P**

(62) Document number(s) of the earlier application(s) in accordance with Art. 76 EPC:
**05822928.7 / 1 831 243**

(71) Applicant: **Novo Nordisk A/S**
**2880 Bagsværd (DK)**

(72) Inventors:
- **Johannsen, Ib**
  **3500 Værløse (DK)**
- **Gallego, Monica Ramos**
  **4320 Lejre (DK)**
- **Roice, Michael**
  **2000 Frederiksberg C (DK)**
- **Nothelfer, Franz**
  **88400 Biberach (DE)**
- **Ambrosius, Dorothee**
  **88471 Laupheim (DE)**
- **Jacobi, Alexander**
  **88471 Laupheim (DE)**

(74) Representative: **Inspicos A/S**
**Kogle Allé 2**
**P.O. Box 45**
**2970 Hørsholm (DK)**

Remarks:
This application was filed on 21-10-2009 as a divisional application to the application mentioned under INID code 62.

(54) **Antibody binding affinity ligands**

(57) The present application discloses a solid support material having covalently immobilized thereon an affinity ligand, said ligand comprising one or more hydrophobic functional group(s) and one or more cationic functional group(s) or one or more heteroaromatic functional group(s), wherein at least one hydrophobic functional group is separated from at least one cationic/heteroaromatic functional group by a through bond distance of from 5 Å to 20 Å, wherein said ligand has a molecular weight of from 120 Da to 5,000 Da. Typically, the affinity resin has a binding capacity larger than 5 mg monoclonal antibody per mL of affinity resin. A method for the isolation of biomolecules, such as proteins, in particular antibodies, such as monoclonal antibodies, or derivatives thereof, is also disclosed.

FIGURE 1

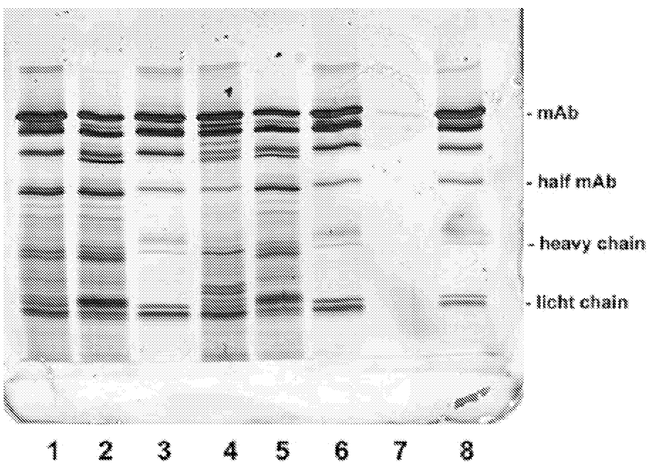




Printed by Jouve, 75001 PARIS (FR)

**Description**

FIELD OF THE INVENTION

The present invention relates to affinity ligands covalently bound to a solid support material, such as a polymer matrix, and uses thereof in the purification and/or isolation of biomolecules, such as proteins, in particular antibodies, such as monoclonal antibodies. The affinity ligands comprise two different domains or functional groups: (i) a hydrophobic domain and (ii) a cationic domain.

BACKGROUND OF THE INVENTION

Affinity chromatography enables selectively and reversibly adsorbing biological substances, such as monoclonal antibodies, to a complementary binding substance, such as an affinity ligand immobilised on a solid support material packed in an affinity column.

Affinity columns often contain a solid support material, usually a porous, polymer matrix, to which a suitable ligand is covalently attached directly or by means of a linker. A sample containing biological substances having an affinity for the ligand can be brought into contact with the affinity ligand covalently immobilised to the solid support material under suitable binding conditions which promote a specific binding between the ligand and the biological substances having an affinity for the ligand. The column can subsequently be washed with a buffer to remove unbound material, and in a further step biological substances having an affinity for the ligand can be eluted and obtained in a purified or isolated form. Accordingly, the ligand should preferably exhibit specific and reversible binding characteristics to the biological substance, such as an antibody, which is desired to purify or isolate.

Antibodies have one or more copies of a Y-shaped unit, composed of four polypeptide chains. Each Y contains two identical copies of a "heavy" chain, and two identical copies of a "light chain", named as such by their relative molecular weights.

Antibodies can be divided into five classes: IgG, IgM, IgA, IgD and IgE, based on the number of Y units and the type of heavy chain. The heavy chain determines the subclass of each antibody. Heavy chains of IgG, IgM, IgA, IgD, and IgE are known as gamma, mu, alpha, delta, and epsilon, respectively. The light chains of any antibody can be classified as either a kappa (κ) or lambda (λ) type (a description of molecular characteristics of the polypeptide).

For pharmaceutical applications, the most commonly used antibody is IgG which can be cleaved into three parts, two F(ab) regions and one Fc region, by the proteolytic enzyme papain, or into two parts, one $F(ab')_2$ and one Fc region by the proteolytic enzyme pepsin.

The F(ab) regions comprise the "arms" of the antibody, which are critical for antigen binding. The Fc region comprises the "tail" of the antibody and plays a role in immune response, as well as serving as a useful "handle" for manipulating the antibody during some immunochemical procedures. The number of F(ab) regions on the antibody, corresponds with its subclass, and determines the "valency" of the antibody (loosely stated, the number of "arms" with which the antibody may bind its antigen).

The term "antibody" means an immunoglobulin, whether natural or wholly or partially synthetically produced. All fragments and derivatives thereof which maintain specific binding ability are also included in the term. Typical fragments are FC, FAB, heavy chain, and light chain. The term also covers any polypeptide having a binding domain which is homologous or largely homologous, such as at least 95% identical when comparing the amino acid sequence, to an immunoglobulin binding domain. These polypeptides may be derived from natural sources, or partly or wholly synthetically produced. An antibody may be monoclonal or polyclonal. The antibody may be a member of any immunoglobulin class, including any of the human classes: IgG, IgM, IgA, IgD, and IgE. Derivatives of the IgG class, however, are preferred in one embodiment of the present invention.

The term "antibody fragment" refers to any derivative of an antibody which is less than full-length. Preferably, the antibody fragment retains at least a significant portion of the specific binding ability of the full-length antibody. Examples of antibody fragments include, but are not limited to, Fab, Fab', $F(ab')_2$, scFv, Fv, dsFv diabody, and Fd fragments. The antibody fragment may be produced by any means. For instance, the antibody fragment may be enzymatically or chemically produced by fragmentation of an intact antibody or it may be recombinantly produced from a gene encoding the partial antibody sequence. Alternatively, the antibody fragment may be wholly or partially synthetically produced. The antibody fragment may optionally be a single chain antibody fragment. Alternatively, the fragment may comprise multiple chains which are linked together, for instance, by disulfide linkages. The fragment may also optionally be a multimolecular complex. A functional antibody fragment will typically comprise at least about 50 amino acids and more typically will comprise at least about 200 amino acids.

"Single-chain Fvs" (scFvs) are recombinant antibody fragments consisting of only the variable light chain ($V_L$) and variable heavy chain ($V_H$) covalently connected to one another by a polypeptide linker. Either $V_L$ or $V_H$ may be the amino-terminal domain. The polypeptide linker may be of variable length and composition so long as the two variable

domains are bridged without serious steric interference. Typically, the linkers are comprised primarily of stretches of glycine and serine residues with some glutamic acid or lysine residues interspersed for solubility. "Diabodies" are dimeric scFvs. The components of diabodies typically have shorter peptide linkers than most scFvs and they show a preference for associating as dimers. An "Fv" fragment is an antibody fragment which consists of one $V_H$ and one $V_L$ domain held together by non-covalent interactions. The term "dsFv" is used herein to refer to an Fv with an engineered intermolecular disulfide bond to stabilize the $V_H$- $V_L$ pair. A "$F(ab')_2$" fragment is an antibody fragment essentially equivalent to that obtained from immunoglobulins (typically IgG) by digestion with an enzyme pepsin at pH 4.0-4.5. The fragment may be recombinantly produced. A "Fab'" fragment is an antibody fragment essentially equivalent to that obtained by reduction of the disulfide bridge or bridges joining the two heavy chain pieces in the $F(ab')_2$ fragment. The Fab' fragment may be recombinantly produced. A "Fab" fragment is an antibody fragment essentially equivalent to that obtained by digestion of immunoglobulins (typically IgG) with the enzyme papain. The Fab fragment may be recombinantly produced. The heavy chain segment of the Fab fragment is the Fd piece. A "Fc" region is a constant region of a particular class of antibody.

The bonding between antigens and antibodies is dependent on hydrogen bonds, hydrophobic bonds, electrostatic forces, and van der Waals forces. These are all bonds of a weak, non-covalent nature, yet some associations between an antigen and an antibody can be quite strong. Accordingly, the affinity constant for antibody-antigen binding can span a wide range, extending from below $10^5$ $mol^{-1}$ to more than $10^{12}$ $mol^{-1}$. Affinity constants are affected by temperature, pH and solvent. Apart from an affinity of an antibody for a ligand, the overall stability of an antibody-ligand complex is also determined by the valency of the antigen and antibody and the structural arrangement of the interacting parts.

Accurate affinity constants can only be determined for monoclonal antibodies which are genetically identical molecules recognising one single epitope on the antigen whereas for polyclonal antibodies a broad distribution of affinities may contribute to an apparent affinity constant. The apparent affinity constant may also be caused by the fact that polyclonal antibodies may recognise more than one single epitope on the same antigen. Since antibodies normally harbour more than one binding domain per molecule multiple, cooperative bondings take place between antibodies and their antigens; this effect is termed avidity. As monoclonal antibodies react with only one single epitope on the antigen they are more vulnerable to the loss of epitope through chemical treatment of the antigen than polyclonal antibodies. This can be offset by pooling two or more monoclonal antibodies to the same antigen.

Monoclonal antibodies can be raised by fusion of B lymphocytes with immortal cell cultures to produce hybridomas. Hybridomas will produce many copies of the exact same antibody - an essential feature in the development of antibodies for therapeutic or diagnostic applications.

Currently, the most explored affinity ligand for the purification and isolation of biomolecules, such as monoclonal antibodies is Protein A. Protein A is a widely used ligand, however, the ligand may suffer from several shortcomings, such as problems relating to instability concomitant with leaching from the column and the need to remove it from the final product or insufficient sanitation of the chromatography resin and moreover, Protein A is fairly expensive.

Hence, there exists a need for novel, stable, inexpensive ligands for isolating antibodies, in particular monoclonal antibodies, or analogues, derivatives, fragments and precursors thereof, whether derived from natural or recombinant sources.

## SUMMARY OF THE INVENTION

In one aspect of the present invention, there is provided a solid support material having covalently immobilized thereon an affinity ligand, said ligand comprising one or more hydrophobic functional group(s) and one or more cationic functional group(s),

wherein at least one hydrophobic functional group is separated from at least one cationic functional group by a through bond distance of from 5 Å, to 20 Å,

wherein said ligand has a molecular weight of from 120 Da to 5,000 Da.

In another aspect of the present invention, there is provided a solid support material having covalently immobilized thereon an affinity ligand, said ligand having one or more hydrophobic functional group(s) and one or more heteroaromatic functional group(s),

wherein at least one hydrophobic functional group is separated from at least one heteroaromatic functional group by a through bond distance of from 5 Å, to 20 Å, and

wherein said ligand has a molecular weight of from 120 Da to 5,000 Da.

In a further aspect of the present invention, there is provided a method for the isolation of biomolecules, such as proteins, e.g. antibodies, in particular monoclonal antibodies, or derivatives thereof, the method comprising the steps of (i) providing a solid support material having covalently immobilized thereon an affinity ligand as defined herein, (ii) providing a sample putatively containing an antibody having an affinity for said ligand, (iii) contacting said ligand with said sample putatively containing said antibody, (iv) binding selectively said antibody when said antibody is contained in said sample and (v) isolating selectively said antibody when said antibody is contained in said sample.

BRIEF DESCRIPTION OF THE FIGURES

Figure 1: Resin B2 selectivity analysis. 1 = fermentation supernatant, 2 = flow through (cycle 1), 3 = elution (cycle 1), 4 = regeneration/sanitation (cycle 1), 5 = washing (cycle 2), 6 = elution (cycle 2), 7 = no protein, 8 = mAb reference sample.

Figure 2: Resin B3 selectivity analysis. 1 = fermentation supernatant, 2 = flow through (cycle 1), 3 = elution (cycle 1), 4 = regeneration/sanitation (cycle 1), 5 = washing (cycle 2), 6 = elution (cycle 2), 7 = regeneration/sanitation (cycle 2), 8 = mAb reference sample.

Figure 3: Resin D1 selectivity analysis. 1 = mAb reference sample, 2 = fermentation supernatant, 3 = flow through (cycle 1), 4 = elution (cycle 1), 5 = regeneration/sanitation (cycle 1), 6 = washing (cycle 2), 7 = elution (cycle 2), 8 = regeneration/sanitation (cycle 2).

Figure 4: Resin D2 selectivity analysis. 1 = mAb reference sample, 2 = fermentation supernatant, 3 = flow through (cycle 1), 4 = elution (cycle 1), 5 = regeneration/sanitation (cycle 1), 6 = washing (cycle 2), 7 = elution (cycle 2), 8 = regeneration/sanitation (cycle 2).

DETAILED DISCLOSURE OF THE INVENTION

As mentioned above, the present invention relates to novel solid support materials having covalently immobilised thereon an affinity ligand, wherein the ligand comprises a particular set of functional groups. Such materials are particularly useful for the purification and isolation of biomolecules, such as proteins, e.g. antibodies, in particular monoclonal antibodies, or derivatives thereof.

*Ligands*

When used herein, the term "ligand" means a molecule which can bind a target compound, for example an antibody, in particular a monoclonal antibody. Ligands preferably bind their binding partners at least in a substantially specific manner ("specific binding").

"Specific binding" refers to the property of a ligand to: (1) bind to a binding partner, e.g. a monoclonal antibody, (2) preferentially such that the relative mass of bound binding partner e.g., a monoclonal antibody, is at least two-fold, such as 50-fold, for example 100-fold, such as 1000-fold, or more greater than the relative mass of other bound species than the binding partner, e.g. a monoclonal antibody. By relative mass of bound compound is meant the mass of bound compound minus the mass of unbound compound divided by the total mass of binding partner, i.e.

$$\text{relative mass of bound compound} = (\text{mass of bound compound} - \text{mass of unbound compound})/(\text{mass of bound compound} + \text{mass of unbound compound}),$$

compound being binding partner or other species.

The term "binding partner" means any biological molecule which is bound by a particular ligand, preferably in a substantially specific manner. A binding partner may be shared by more than one ligand. Preferred binding partners are antibodies including polyclonal antibodies and monoclonal antibodies. Further preferred binding partners are antibody fragments from monoclonal antibodies or polyclonal antibodies.

The ligands according to the invention include enriched or resolved optical isomers at any or all asymmetric atoms as are apparent from the description or depiction herein. Both racemic and diasteromeric mixtures, as well as the individual optical isomers can be isolated or synthesized so as to be substantially free of their enantiomeric or diastereomeric partners, and these are all within the scope of the invention.

Experiments have surprisingly shown that certain classes of affinity ligands, i.a. one where the ligands comprise one or more hydrophobic functional group(s) and one or more cationic functional group(s) bind selectively to mAbs. Another promising class of affinity ligands is the one where the ligands comprise one or more hydrophobic functional group(s) and one or more heteroaromatic functional group(s).

It was further found that the at least one hydrophobic functional group preferably should be separated from the

at least one cationic functional group by a through bond distance of from 5 Å, to 30 Å, for example a through bond distance of from 5 Å to 20 Å, such as a through bond distance of from 5 to 19 Å, for example a through bond distance of from 5 to 18 Å, such as a through bond distance of from 5 to 17 Å, for example a through bond distance of from 5 to 16 Å, such as a through bond distance of from 5 to 15 Å, for example a through bond distance of from 5 to 14 Å, such as a through bond distance of from 5 to 13 Å, for example a through bond distance of from 5 to 12 Å, such as a through bond distance of from 5 to 11 Å, for example a through bond distance of from 5 to 10 Å, such as a through bond distance of from 6 to 14 Å, or such as a through bond distance of from 7 to 20 Å, for example a through bond distance of from 7 to 19 Å, such as a through bond distance of from 7 to 18 Å, for example a through bond distance of from 7 to 17 Å, such as a through bond distance of from 7 to 16 Å, for example a through bond distance of from 7 to 15 Å, such as a through bond distance of from 7 to 14 Å, for example a through bond distance of from 7 to 13 Å, such as a through bond distance of from 7 to 12 Å, for example a through bond distance of from 7 to 11 Å, such as a through bond distance of from 7 to 10 Å, for example a through bond distance of from 8 to 12 Å, or for example a through bond distance of from 9 to 20 Å, such as a through bond distance of from 9 to 18 Å, for example a through bond distance of from 9 to 16 Å, such as a through bond distance of from 9 to 14 Å, for example a through bond distance of from 9 to 12 Å, such as a through bond distance of from 9 to 11 Å, for example a through bond distance of about 10 Å.

Through bond distance is the shortest intra molecular through bond distance between covalently linked chemical entities along the chemical bonds. It is calculated by adding the individual atom-atom bond distances along the shortest intramolecular path. Typical atom-atom bond distances are 1.2 Å to 1.4 Å.

The distance <u>through space</u> between the at least one hydrophobic functional group and the at least one cationic functional group of the ligand is preferably less than 30 Å, such as less than 28 Å, for example less than 26 Å, such as less than 24 Å, for example less than 22 Å, such as less than 20 Å, for example less than 18 Å, such as about or less than 16 Å, for example less than 15 Å, such as about or less than 14 Å, for example less than 13 Å, such as about or less than 12 Å, for example less than 11 Å, such as about or less than 10 Å, four example about or less than 8 Å, such as about 6 Å, for example in the range of from 5 to 20 Å, such as a distance through space of from 5 to 19 Å, for example a distance through space of from 5 to 18 Å, such as a distance through space of from 5 to 17 Å, for example a distance through space of from 5 to 16 Å, such as a distance through space of from 5 to 15 Å, for example a distance through space of from 5 to 14 Å, such as a distance through space of from 5 to 13 Å, for example a distance through space of from 5 to 12 Å, such as a distance through space of from 5 to 11 Å, for example a distance through space of from 5 to 10 Å, such as a distance through space of from 7 to 20 Å, for example a distance through space of from 7 to 19 Å, such as a distance through space of from 7 to 18 Å, for example a distance through space of from 7 to 17 Å, such as a distance through space of from 7 to 16 Å, for example a distance through space of from 7 to 15 Å, such as a distance through space of from 7 to 14 Å, for example a distance through space of from 7 to 13 Å, such as a distance through space of from 7 to 12 Å, for example a distance through space of from 7 to 11 Å, such as a distance through space of from 7 to 10 Å, for example a distance through space of from 9 to 20 Å, such as a distance through space of from 9 to 18 Å, for example a distance through space of from 9 to 16 Å, such as a distance through space of from 9 to 14 Å, for example a distance through space of from 9 to 12 Å, such as a distance through space of from 9 to 11 Å.

Through bond distances and distances through space can be calculated or determined by the person skilled in the art according to state of the art techniques. Molecular modelling can also be used for determining the minimum distance between atoms of different ligand functional groups. Molecular modelling can be performed e.g. with Sybyl/Mendyl 5.4 (Tripos Associates, St. Louis, Mo.) using an Evans and Sutherland PS390 graphics computer equipped with a stereographic viewer. Structures of suitable ligands can be provided via construction with the Concord program or from libraries followed by energy minimization. Energy calculations can be made with Sybyl/Mendyl force field and a 1.2 Å van der Waals parameter for hydrogen. Charges can be calculated using the Gasteigner-Huckel method which includes sigma-bonding and pi-bonding.

The ligand preferably has a molecular weight of less than 5,000 Da, such as less than 4,000 Da, for example less than 3,500 Da, such as less than 3,000 Da, for example less than 2,500 Da, such as less than 2,000 Da, such as less than 1,800 Da, for example less than 1,600 Da, such as less than 1,500 Da, such as less than 1,400 Da, for example less than 1,300 Da, such as less than 1,200 Da, such as less than 1,100 Da, for example less than 1,000 Da.

Additionally, the ligand preferably has a molecular weight of more than 120 Da, such as more than 140 Da, for example more than 160 Da, such as more than 180 Da, for example more than 200 Da, such as more than 220 Da, for example a molecular weight of more than 240 Da.

In order to reduce the degree of non-specific binding to the cationic groups, the ligand may further comprise one or more anionic groups in order to compensate some of the positive charge on the ligand. The anionic groups include, but are not limited to, carboxylate, sulfonate, sulphate, phosphate and other negatively charged ionisable groupings, and can e.g. be disposed upon groups pendant from the ligand.

*Ligand functional groups*

In one main group of suitable ligands, each ligand comprises one or more hydrophobic functional group(s) and one or more cationic functional group(s).
In another main group of suitable ligands, each ligand comprises one or more hydrophobic functional group(s) and one or more heteroaromatic functional group(s).
As used herein, a cationic functional group is an organic group which has a positive charge in the pH range 3-7. Primary, secondary, and tertiary amines are typical examples of cationic groups. Guanidine is a further relevant example. Further examples of cationic functional groups are given in the section "Cationic functional groups" below.
A hydrophobic functional group is an organic group capable of binding to the surface of a biomolecule mainly by hydrophobic interaction. Hydrophobic functional groups are characterised by being essentially non-polar and uncharged at normal physiological conditions. Hydrophobic residues are repelled by aqueous solution so as to seek the inner positions in the conformation of a ligand when the ligand is in an aqueous medium. Also, hydrophobic residues will seek towards hydrophobic pockets or grooves of ligand binding partners when the ligand is associated with a binding partner under normal physiological conditions.
When used herein, the term "normal physiological condition" means conditions that are typical inside a living organism or a cell. While it is recognized that some organs or organisms provide extreme conditions, the intra-organismal and intra-cellular environment normally varies around pH 7 (i.e., from pH 6.5 to pH 7.5), contains water as the predominant solvent, and exists at a temperature above 0°C and below 50°C. It will be recognized that the concentration of various salts depends on the organ, organism, cell, or cellular compartment used as a reference.
Organic hydrophobic groups generally have a high content of carbon atoms. Typical examples of hydrophobic groups are linear and branched alkanes, cyclic hydrocarbons, aromatic compounds, and combinations of linear and branched alkanes, cyclic hydrocarbons, and aromatic compounds. Also substituted variants of such groups are considered as being hydrophobic as long as the relative content of carbon is above a certain limit. However, the percentage of carbon atoms is not the only parameter, which influences the hydrophobicity. Also the position and nature of other atoms play an important role. E.g. an ether is typically more hydrophobic than an alcohol with the same number of carbon atoms and oxygen atoms, and an ester is more hydrophobic than a di-ol with the same elemental composition. When one or more possible non-carbon and non-hydrogen atoms of an organic group are at primary positions the relative number of carbon atoms must be higher for the group to be hydrophobic than when possible non-carbon and non-hydrogen atoms are at secondary, tertiary, or quarternary positions. Keeping this in mind, we define a hydrophobic group as an organic groups with 75% or more of its non-hydrogen atoms being carbon atoms, such as 80% or more, preferably 85% or more of its non-hydrogen atoms being carbon atoms. E.g. the lower value, 75%, applies to ethers and esters, the intermediate value, 80%, applies to amides and secondary and tertiary amines, whereas the upper value, 85%, applies to alcohols and primary amines. Examples of hydrophobic functional groups are given in the section 'Ligand functional groups' below.
The at least one hydrophobic functional group can comprise or consist of one or more groups selected from "alkyl", "cyclic alkyl", "substituted alkyl", "aryl", "substituted aryl", "alkenyl", "substituted alkenyl", "alkynyl", "substituted alkynyl", "aralkyl", "substituted aralkyl", "heterocyclyl", "substituted heterocyclyl", "heterocyclylalkyl", "substituted heterocyclylalkyl", "alkylaminoalkyl", "substituted alkylaminoalkyl", "dialkylaminoalkyl", "substituted dialkylaminoalkyl", "heterocyclyloxyalkyl", "substituted heterocyclyloxyalkyl", "arylaminoalkyl", "substituted arylaminoalkyl", "heterocyclylaminoalkyl", "substituted heterocyclylaminoalkyl", "alkylaminoalkoxy", "substituted alkylaminoalkoxy", "dialkylaminoalkoxy", "substituted dialkylaminoalkoxy", "heterocyclyloxy", and "substituted heterocyclyloxy", as defined herein below.

<u>Hydrophobic functional groups comprising aliphatic residues</u>

The at least one hydrophobic functional group can comprise or consists of an optionally substituted aliphatic residue and/or an optionally substituted aromatic residue. Aliphatic residues generally refer to hydrocarbons such as e.g. alkyl, alkylene and alkynyl residues which can be substituted or non-substituted.
"Alkyl" as used herein includes straight chain alkyl groups such as methyl, ethyl, propyl, butyl, pentyl, hexyl, heptyl, octyl, nonyl, decyl, undecyl, dodecyl and the like. "Alkyl" also includes branched chain isomers of straight chain alkyl groups, including but not limited to, the following which are provided by way of example: $-CH(CH_3)_2$, $-CH(CH_3)(CH_2CH_3)$, $-CH(CH_2CH_3)_2$, $-C(CH_3)_3$, $-C(CH_2CH_3)_3$, $-CH_2CH(CH_3)_2$, $-CH_2CH(CH_3)(CH_2CH_3)$, $-CH_2CH(CH_2CH_3)_2$, $-CH_2C(CH_3)_3$, $-CH_2C(CH_2CH_3)_3$, $-CH(CH_3)CH(CH_3)(CH_2CH_3)$, $-CH_2CH_2CH(CH_3)_2$, $-CH_2CH_2CH(CH_3)(CH_2CH_3)$, $-CH_2CH_2CH(CH_2CH_3)_2$, $-CH_2CH_2C(CH_3)_3$, $-CH_2CH_2C(CH_2CH_3)_3$, $-CH(CH_3)CH_2CH(CH_3)_2$, $-CH(CH_3)CH(CH_3)CH(CH_3)CH(CH_3)_2$, $-CH(CH_2CH_3)CH(CH_3)CH(CH_3)(CH_2CH_3)$, and others.
The aliphatic residue can be an optionally substituted linear aliphatic residue or an optionally substituted branched aliphatic residue. The aliphatic residue can also be an optionally substituted cyclic alkyl. "Cyclic alkyl" includes groups such as cyclopropyl, cyclobutyl, cyclopentyl, cyclohexyl, cycloheptyl, and cyclooctyl and such rings substituted

with straight and branched chain alkyl groups as defined above, and also includes polycyclic alkyl groups such as, but not limited to, adamantyl norbornyl, and bicyclo[2.2.2]octyl and such rings substituted with straight and branched chain alkyl groups as defined above. Thus, unsubstituted alkyl groups include primary alkyl groups, secondary alkyl groups, and tertiary alkyl groups. Unsubstituted alkyl groups may be bonded to one or more carbon atom(s), oxygen atom(s), nitrogen atom(s), and/or sulfur atom(s) in a ligand.

The cyclic aliphatic residue can e.g. comprise or consist of a $C_5$-$C_{16}$ cycloalkyl group. Shorter chain lengths can also occur, typically when the cycloalkyl is substituted with an aryl or heteroaryl residue.

"Substituted alkyl" refers to an unsubstituted alkyl group as defined above in which one or more bonds to a carbon(s) or hydrogen(s) are replaced by a bond to non-hydrogen and non-carbon atoms such as, but not limited to, a halogen atom in halides such as F, Cl, Br, and I; and oxygen atom in groups such as hydroxyl groups, alkoxy groups, aryloxy groups, and ester groups; a sulfur atom in groups such as thiol groups, alkyl and aryl sulfide groups, sulfone groups, sulfonyl groups, and sulfoxide groups; a nitrogen atom in groups such as amines, amides, alkylamines, dialkylamines, arylamines, alkylarylamines, diarylamines, N-oxides, imides, and enamines; a silicon atom in groups such as in trialkylsilyl groups, dialkylarylsilyl groups, alkyldiarylsilyl groups, and triarylsilyl groups; and other heteroatoms in various other groups.

Substituted alkyl groups also include groups in which one or more bonds to a carbon(s) or hydrogen(s) atom is replaced by a bond to a heteroatom such as oxygen in carbonyl, carboxyl, and ester groups; nitrogen in groups such as imines, oximes, hydrazones, and nitriles. Substituted alkyl groups also include, among others, alkyl groups in which one or more bonds to a carbon or hydrogen atom is/are replaced by one or more bonds to a halogen atom. Other substituted alkyl groups include those in which one or more bonds to a carbon or hydrogen atom is replaced by a bond to an oxygen atom such that the substituted alkyl group contains a hydroxyl, alkoxy, aryloxy group, or heterocyclyloxy group. Still other alkyl groups include alkyl groups that have an amine, alkylamine, dialkylamine, arylamine, (alkyl)(aryl) amine, diarylamine, heterocyclylamine, (alkyl)(heterocyclyl)amine, (aryl)(heterocyclyl)amine, or diheterocyclylamine group.

In one embodiment, an aliphatic functional group is preferably substituted with an aryl group such as an ($C_6$-$C_{12}$) aryl group mentioned herein below, which may in turn also be substituted, as also described herein. An example of a substituted aryl group includes an "aralkyl group", which can be substituted or non-substituted.

Accordingly, "aralkyl" refers to unsubstituted alkyl groups as defined above in which a hydrogen or carbon bond of the unsubstituted alkyl group is replaced with a bond to an aryl group as defined above. For example, methyl ($-CH_3$) is an unsubstituted alkyl group. If a hydrogen atom of the methyl group is replaced by a bond to a phenyl group, such as if the carbon of the methyl was bonded to a carbon of benzene, then the compound is an unsubstituted aralkyl group (i.e., a benzyl group). Thus includes, but is not limited to, groups such as benzyl, diphenylmethyl, and 1-phenylethyl ($-CH(C_6H_5)(CH_3)$), 2-phenylethyl group, 2-naphthylethyl group, and the like.

"Substituted aralkyl" has the same meaning with respect to unsubstituted aralkyl groups that substituted aryl groups had with respect to unsubstituted aryl groups. However, a substituted aralkyl group also includes groups in which a carbon or hydrogen bond of the alkyl part of the group is replaced by a bond to a non-carbon or a non-hydrogen atom. Examples of substituted aralkyl groups include, but are not limited to, $-CH_2C(=O)(C_6H_5)$, and $-CH_2$(2-methylphenyl) among others.

In one embodiment, the optionally substituted aliphatic residue comprises or consists of a $C_5$-$C_{20}$ alkyl group. Shorter chain lengths can also occur, typically when the alkyl is substituted with an aryl or heteroaryl residue. Further examples of alkyl groups substituted with aryl or heteroaryl include, for example, a linear ($C_1$-$C_{10}$), branched ($C_4$-$C_{10}$) or cyclic ($C_5$-$C_{10}$) group, such as a methyl group, ethyl group, propyl group, such as a n-propyl group and an isopropyl group, butyl group, such as n-butyl group, isobutyl group, t-butyl group, n-amyl group, pentyl group, such as neopentyl group, cyclopentyl group, hexyl group, such as n-hexyl group, cyclohexyl group, heptyl group, octyl group, such as n-octyl group, nonyl group, such as n-nonyl group, decyl group, such as n-decyl group, undecyl group, dodecyl group, mentyl group, 2,3,4-trimethyl-3-pentyl group, 2,4-dimethyl-3-pentyl group, and the like.

In one embodiment, a $C_5$-$C_{20}$ alkyl group can also be substituted, for example with a halogen atom, an alkoxy group, an aryloxy group, an alkylthio group, or an arylthio group. Examples of the halogen atom are a fluorine atom, a chlorine atom, a bromine atom, and an iodine atom. Examples of the alkoxyl group include, for example, a ($C_1$-$C_4$) alkoxy group such as methoxy group, ethoxy group, n-propoxy group, t-butoxy group or the like. Examples of the alkylthio group include, for example, those comprised of the ($C_1$-$C_{10}$) alkyl group, as described above, and thio group, and specific examples thereof include, for example, n-propylthio group, t-butylthio group or the like. Examples of the arylthio group include, for example, those comprised of the ($C_6$-$C_{12}$) aryl group, as described above, and a thio group, and specific examples thereof include, for example, a phenylthio group or the like. Examples of the aryloxy group, which may be present on the aryl, heteroaryl, and saturated hydrocarbon groups, for example, those comprised of the ($C_6$-$C_{12}$) aryl group, as described above, and an oxy group, and specific examples thereof include, for example, a phenoxy group or the like.

The alkyl groups described herein above can contain one or more carbon-carbon double bonds (alkenyl groups)

or one or more carbon-carbon triple bonds (alkynyl groups).

"Alkenyl" refers to straight and branched chain and cyclic groups such as those described with respect to unsubstituted alkyl groups as defined above, except that at least one double bond exists between two carbon atoms. Examples include, but are not limited to vinyl, - $CH=C(H)(CH_3)$, $-CH=C(CH_3)_2$, $-C(CH_3)=C(H)_2$, $-C(CH_3)=C(H)(CH_3)$, -C ($CH_2$ $CH_3$)=$CH_2$, cyclohexenyl, cyclopentenyl, cyclohexadienyl, butadienyl, pentadienyl, and hexadienyl among others.

"Substituted alkenyl" has the same meaning with respect to unsubstituted alkenyl groups that substituted alkyl groups had with respect to unsubstituted alkyl groups. A substituted alkenyl group includes alkenyl groups in which a non-carbon or non-hydrogen atom is bonded to a carbon double bonded to another carbon and those in which one of the non-carbon or non-hydrogen atoms is bonded to a carbon not involved in a double bond to another carbon.

"Alkynyl" refers to straight and branched chain groups such as those described with respect to alkyl groups as defined above, except that at least one triple bond exists between two carbon atoms. Examples include, but are not limited to -CC(H), $-CC(CH_3)$, $-CC(CH_2CH_3)$, - $C(H_2)CC(H)$, $-C(H)_2CC(CH_3)$, and $-C(H)_2CC(CH_2CH_3)$ among others.

"Substituted alkynyl" has the same meaning with respect to unsubstituted alkynyl groups that substituted alkyl groups had with respect to unsubstituted alkyl groups. A substituted alkynyl group includes alkynyl groups in which a non-carbon or non-hydrogen atom is bonded to a carbon triple bonded to another carbon and those in which a non-carbon or non-hydrogen atom is bonded to a carbon not involved in a triple bond to another carbon.

Further examples of substituted alkyl groups are described herein below.

"Alkylaminoalkyl" refers to an unsubstituted alkyl group as defined above in which a carbon or hydrogen bond is replaced by a bond to a nitrogen atom that is bonded to a hydrogen atom and an unsubstituted alkyl group as defined above. For example, methyl ($-CH_3$) is an unsubstituted alkyl group. If a hydrogen atom of the methyl group is replaced by a bond to a nitrogen atom that is bonded to a hydrogen atom and an ethyl group, then the resulting compound is $-CH_2-N(H)(CH_2CH_3)$ which is an unsubstituted alkylaminoalkyl group. "Substituted alkylaminoalkyl" refers to an unsubstituted alkylaminoalkyl group as defined above except where one or more bonds to a carbon or hydrogen atom in one or both of the alkyl groups is replaced by a bond to a non-carbon or non-hydrogen atom as described above with respect to substituted alkyl groups except that the bond to the nitrogen atom in all alkylaminoalkyl groups do not by itself qualify all alkylaminoalkyl groups as being substituted. However, substituted alkylaminoalkyl groups do include groups in which the hydrogen bonded to the nitrogen atom of the group is replaced with a non-carbon and non-hydrogen atom.

"Dialkylaminoalkyl" refers to an unsubstituted alkyl group as defined above in which a carbon bond or hydrogen bond is replaced by a bond to a nitrogen atom which is bonded to two other similar or different unsubstituted alkyl groups as defined above.

"Substituted dialkylaminoalkyl" refers to an unsubstituted dialkylaminoalkyl group as defined above in which one or more bonds to a carbon or hydrogen atom in one or more of the alkyl groups is replaced by a bond to a non-carbon and non-hydrogen atom as described with respect to substituted alkyl groups. The bond to the nitrogen atom in all dialkylaminoalkyl groups do not by itself qualify all dialkylaminoalkyl groups as being substituted.

"Heterocyclyloxyalkyl" refers to an unsubstituted alkyl group as defined above in which a carbon bond or hydrogen bond is replaced by a bond to an oxygen atom which is bonded to an unsubstituted heterocyclyl group as defined above.

"Substituted heterocyclyloxyalkyl" refers to an unsubstituted heterocyclyloxyalkyl group as defined above in which a bond to a carbon or hydrogen group of the alkyl group of the heterocyclyloxyalkyl group is bonded to a non-carbon and non-hydrogen atom as described above with respect to substituted alkyl groups or in which the heterocyclyl group of the heterocyclyloxyalkyl group is a substituted heterocyclyl group as defined above.

"Arylaminoalkyl" refers to an unsubstituted alkyl group as defined above in which a carbon bond or hydrogen bond is replaced by a bond to a nitrogen atom which is bonded to at least one unsubstituted aryl group as defined above.

"Substituted arylaminoalkyl" refers to an unsubstituted arylaminoalkyl group as defined above except where either the alkyl group of the arylaminoalkyl group is a substituted alkyl group as defined above or the aryl group of the arylaminoalkyl group is a substituted aryl group except that the bonds to the nitrogen atom in all arylaminoalkyl groups do not by itself qualify all arylaminoalkyl groups as being substituted. However, substituted arylaminoalkyl groups do include groups in which the hydrogen bonded to the nitrogen atom of the group is replaced with a non-carbon and non-hydrogen atom.

"Heterocyclylaminoalkyl" refers to an unsubstituted alkyl group as defined above in which a carbon or hydrogen bond is replaced by a bond to a nitrogen atom which is bonded to at least one unsubstituted heterocyclyl group as defined above.

"Substituted heterocyclylaminoalkyl" refers to unsubstituted heterocyclylaminoalkyl groups as defined above in which the heterocyclyl group is a substituted heterocyclyl group as defined above and/or the alkyl group is a substituted alkyl group as defined above. The bonds to the nitrogen atom in all heterocyclylaminoalkyl groups do not by itself qualify all heterocyclylaminoalkyl groups as being substituted. However, substituted heterocyclylaminoalkyl groups do include groups in which the hydrogen bonded to the nitrogen atom of the group is replaced with a non-carbon and non-hydrogen atom.

"Alkylaminoalkoxy" refers to an unsubstituted alkyl group as defined above in which a carbon or hydrogen bond is replaced by a bond to an oxygen atom which is bonded to the parent compound and in which another carbon or hydrogen bond of the unsubstituted alkyl group is bonded to a nitrogen atom which is bonded to a hydrogen atom and an unsubstituted alkyl group as defined above.

"Substituted alkylaminoalkoxy" refers to unsubstituted alkylaminoalkoxy groups as defined above in which a bond to a carbon or hydrogen atom of the alkyl group bonded to the oxygen atom which is bonded to the parent compound is replaced by one or more bonds to a non-carbon and non-hydrogen atoms as discussed above with respect to substituted alkyl groups and/or if the hydrogen bonded to the amino group is bonded to a non-carbon and non-hydrogen atom and/or if the alkyl group bonded to the nitrogen of the amine is bonded to a non-carbon and non-hydrogen atom as described above with respect to substituted alkyl groups. The presence of the amine and alkoxy functionality in all alkylaminoalkoxy groups do not by itself qualify all such groups as substituted alkylaminoalkoxy groups.

"Unsubstituted dialkylaminoalkoxy" refers to an unsubstituted alkyl group as defined above in which a carbon or hydrogen bond is replaced by a bond to an oxygen atom which is bonded to the parent compound and in which another carbon or hydrogen bond of the unsubstituted alkyl group is bonded to a nitrogen atom which is bonded to two other similar or different unsubstituted alkyl groups as defined above.

"Substituted dialkylaminoalkoxy" refers to an unsubstituted dialkylaminoalkoxy group as defined above in which a bond to a carbon or hydrogen atom of the alkyl group bonded to the oxygen atom which is bonded to the parent compound is replaced by one or more bonds to a non-carbon and non-hydrogen atoms as discussed above with respect to substituted alkyl groups and/or if one or more of the alkyl groups bonded to the nitrogen of the amine is bonded to a non-carbon and non-hydrogen atom as described above with respect to substituted alkyl groups. The presence of the amine and alkoxy functionality in all dialkylaminoalkoxy groups do not by itself qualify all such groups as substituted dialkylaminoalkoxy groups.

"Heterocyclyloxy" refers to a hydroxyl group (-OH) in which the bond to the hydrogen atom is replaced by a bond to a ring atom of an otherwise unsubstituted heterocyclyl group as defined above.

"Substituted heterocyclyloxy" refers to a hydroxyl group (-OH) in which the bond to the hydrogen atom is replaced by a bond to a ring atom of an otherwise substituted heterocyclyl group as defined above.

Hydrophobic functional groups comprising aromatic residues

Aromatic residues can be optionally substituted aryl or heteroaryl residues. "Aryl" includes, but is not limited to, groups such as phenyl, biphenyl, anthracenyl, naphthenyl by way of example. Although "aryl" includes groups containing condensed rings such as naphthalene, it does not include aryl groups that have other groups such as alkyl or halo groups bonded to one of the ring members, as aryl groups such as tolyl are considered herein to be substituted aryl groups as described herein below. Aryl groups may be bonded to one or more carbon atom(s), oxygen atom(s), nitrogen atom(s), and/or sulfur atom(s) in the ligand.

"Substituted aryl group" has the same meaning with respect to unsubstituted aryl groups that substituted alkyl groups had with respect to unsubstituted alkyl groups. However, a substituted aryl group also includes aryl groups in which one of the aromatic carbons is bonded to one of the non-carbon or non-hydrogen atoms described above and also includes aryl groups in which one or more aromatic carbons of the aryl group is bonded to a substituted and/or unsubstituted alkyl, alkenyl, or alkynyl group as defined herein. This includes bonding arrangements in which two carbon atoms of an aryl group are bonded to two atoms of an alkyl, alkenyl, or alkynyl group to define a fused ring system (e.g. dihydronaphthyl or tetrahydronaphthyl).

Examples of aryl and heteroaryl include, for example, a ($C_6$-$C_{12}$) aryl group such as a phenyl group, tolyl group, naphthyl group, biphenyl group or the like, and a ($C_4$-$C_5$) heteroaryl group, or pyridyl group, or the like.

Also, when the at least one hydrophobic functional group comprises or consists of an optionally substituted aromatic residue, the aromatic residue can be selected from the group consisting of aromatic residues comprising or consisting of fluorenyl, pyrroyl, furanyl, thienyl, thiophenyl, thiazolyl, isoindolyl, quinolinyl, isoquinolinyl, oxazolyl, and purinyl. Further examples include, but is not limited to tetrahydrothiophenyl, sulfur oxidized tetrahydrothiophenyl, tetrazolyl, benzofuranyl, thianaphthalenyl, indolenyl, benzimidazolyl, piperidinyl, 4-piperidonyl, pyrrolidinyl, 2-pyrrolidonyl, pyrrolinyl, tetrahydrofuranyl, tetrahydroquinolinyl, tetrahydroisoquinolinyl, decahydroquinolinyl, octahydroisoquinolinyl, azocinyl, triazinyl, 6H-1,2,5-thiadiazinyl, 2H,6H-1,5,2-dithiazinyl, thianthrenyl, pyranyl, isobenzofuranyl, chromenyl, xanthenyl, phenoxathiinyl, 2H-pyrrolyl, isothiazolyl, isoxazolyl, pyrazinyl, pyridazinyl, indolizinyl, isoindolyl, 3H-indolyl, 1H-indazoly, 4H-quinolizinyl, phthalazinyl, naphthyridinyl, quinoxalinyl, quinazolinyl, cinnolinyl, pteridinyl, 4aH-carbazolyl, carbazolyl, β-carbolinyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, piperazinyl, indolinyl, quinuclidinyl, morpholinyl, oxazolidinyl, benzotriazolyl, benzisoxazolyl, oxindolyl, benzoxazolinyl, and isatinoyl.

By way of example and not limitation, carbon bonded heterocycles can be bonded at position 2, 3, 4, 5, or 6 of a pyridine, position 3, 4, 5, or 6 of a pyridazine, position 2, 4, 5, or 6 of a pyrimidine, position 2, 3, 5, or 6 of a pyrazine,

position 2, 3, 4, or 5 of a furan, tetrahydrofuran, thiofuran, thiophene, pyrrole or tetrahydropyrrole, position 2, 4, or 5 of an oxazole, imidazole or thiazole, position 3, 4, or 5 of an isoxazole, pyrazole, or isothiazole, position 2 or 3 of an aziridine, position 2, 3, or 4 of an azetidine, position 2, 3, 4, 5, 6, 7, or 8 of a quinoline or position 1, 3, 4, 5, 6, 7, or 8 of an isoquinoline. Still more typically, carbon bonded heterocycles include 2-pyridyl, 3-pyridyl, 4-pyridyl, 5-pyridyl, 6-pyridyl, 3-pyridazinyl, 4-pyridazinyl, 5-pyridazinyl, 6-pyridazinyl, 2-pyrimidinyl, 4-pyrimidinyl, 5-pyrimidinyl, 6-pyrimidinyl, 2-pyrazinyl, 3-pyrazinyl, 5-pyrazinyl, 6-pyrazinyl, 2-thiazolyl, 4-thiazolyl, or 5-thiazolyl.

By way of example and not limitation, nitrogen bonded heterocycles are bonded at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1H-indazole, position 2 of an isoindole, or isoindoline, position 4 of a morpholine, and position 9 of a carbazole, or β-carboline. Typically, nitrogen bonded heterocycles include 1-aziridyl, 1-azetedyl, 1-pyrrolyl, 1-imidazolyl, 1-pyrazolyl, and 1-piperidinyl.

As will be clear from the above, the heteroaromatic group can also be selected from the group consisting of heteroaromatic groups comprising or consisting of optionally substituted, fused heteroaromatic compounds. Examples include e.g. indole, benzothiophene, benzotriazene and quinoline.

In one embodiment, the aromatic residue can be substituted with one or more optionally substituted aliphatic groups, such as the optionally substituted aliphatic groups mentioned herein immediately above, such as the linear, branched or cyclic ($C_1$-$C_{10}$) alkyl group, for example a methyl group, an ethyl group, an isopropyl group, a n-butyl group, a t-butyl group, a n-amyl group, a n-hexyl group and the like.

The aromatic residue can be substituted with one or more heteroatoms, or substituted with one or more aromatic groups, or substituted with one or more heteroaromatic groups.

Also, the aromatic residue can e.g. be substituted with a substituted alkyl or aryl or heteroaryl, wherein the aromatic residue, or the alkyl or the aryl or the heteroaryl is substituted with a heteroatom selected from O, N, S and halogen, or substituted with one or more groups selected from hydroxyl, amino, thiol, halogen, carbonyl, carboxylic acid, ether and ester.

The aryl and heteroaryl groups can also be substituted, for example, with a halogen atom, an alkoxy group, an aryloxy group, an alkylthio group, or an arylthio group.

Examples of the halogen atom are a fluorine atom, a chlorine atom, a bromine atom, and an iodine atom. Examples of the alkoxyl group include, for example, a ($C_1$-$C_4$) alkoxy group such as methoxy group, ethoxy group, n-propoxy group, t-butoxy group or the like. Examples of the alkylthio group include, for example, those comprised of the ($C_1$-$C_{10}$) alkyl group, as described above, and thio group, and specific examples thereof include, for example, n-propylthio group, t-butylthio group or the like. Examples of the arylthio group include, for example, those comprised of the ($C_6$-$C_{12}$) aryl group, as described above, and a thio group, and specific examples thereof include, for example, a phenylthio group or the like. Examples of the aryloxy group, which may be present on the aryl, heteroaryl, and saturated hydrocarbon groups, for example, those comprised of the ($C_6$-$C_{12}$) aryl group, as described above, and an oxy group, and specific examples thereof include, for example, a phenoxy group or the like.

The term "heterocyclyl" as used herein refers to both aromatic and non-aromatic ring compounds including monocyclic, bicyclic, and polycyclic ring compounds such as, but not limited to, quinuclidyl, containing 3 or more ring members of which one or more is a heteroatom such as, but not limited to, N, O, and S. Although "heterocyclyl" includes condensed heterocyclic rings such as benzimidazolyl, it does not include heterocyclyl groups that have other groups such as alkyl or halo groups bonded to one of the ring members as compounds such as 2-methylbenzimidazolyl are substituted heterocyclyl groups. Examples of heterocyclyl groups include, but are not limited to: unsaturated 3 to 8 membered rings containing 1 to 4 nitrogen atoms such as, but not limited to pyrrolyl, pyrrolinyl, imidazolyl, pyrazolyl, pyridyl, dihydropyridyl, pyrimidyl, pyrazinyl, pyridazinyl, triazolyl (e.g. 4H-1,2,4-triazolyl, 1H-1,2,3-triazolyl, 2H-1,2,3-triazolyl etc.), tetrazolyl, (e.g. 1H-tetrazolyl, 2H tetrazolyl, etc.); saturated 3 to 8 membered rings containing 1 to 4 nitrogen atoms such as, but not limited to, pyrrolidinyl, imidazolidinyl, piperidinyl, piperazinyl; condensed unsaturated heterocyclic groups containing 1 to 4 nitrogen atoms such as, but not limited to, indolyl, isoindolyl, indolinyl, indolizinyl, benzimidazolyl, quinolyl, isoquinolyl, indazolyl, benzotriazolyl; unsaturated 3 to 8 membered rings containing 1 to 2 oxygen atoms and 1 to 3 nitrogen atoms such as, but not limited to, oxazolyl, isoxazolyl, oxadiazolyl (e.g. 1,2,4-oxadiazolyl, 1,3,4-oxadiazolyl, 1,2,5-oxadiazolyl, etc.); saturated 3 to 8 membered rings containing 1 to 2 oxygen atoms and 1 to 3 nitrogen atoms such as, but not limited to, morpholinyl; unsaturated condensed heterocyclic groups containing 1 to 2 oxygen atoms and 1 to 3 nitrogen atoms, for example, benzoxazolyl, benzoxadiazolyl, benzoxazinyl (e.g. 2H-1,4-benzoxazinyl etc.); unsaturated 3 to 8 membered rings containing 1 to 3 sulfur atoms and 1 to 3 nitrogen atoms such as, but not limited to, thiazolyl, isothiazolyl, thiadiazolyl (e.g. 1,2,3-thiadiazolyl, 1,2,4-thiadiazolyl, 1,3,4-thiadiazolyl, 1,2,5-thiadiazolyl, etc.); saturated 3 to 8 membered rings containing 1 to 2 sulfur atoms and 1 to 3 nitrogen atoms such as, but not limited to, thiazolodinyl; saturated and unsaturated 3 to 8 membered rings containing 1 to 2 sulfur atoms such as, but not limited to, thienyl, dihydrodithiinyl, dihydrodithionyl, tetrahydrothiophene, tetrahydrothiopyran; unsaturated condensed heterocyclic rings containing 1 to 2 sulfur atoms and 1 to 3 nitrogen atoms such as, but not limited to, benzothiazolyl, benzothiadiazolyl, benzothiazinyl (e.g. 2H-1,4-benzothiazinyl, etc.), dihydrobenzothiazinyl (e.g. 2H-3,4-dihydrobenzothiazinyl, etc.), unsaturated 3 to 8 membered rings containing oxygen atoms such as, but not limited to furyl; unsaturated condensed heterocyclic rings containing 1 to 2 oxygen atoms such as benzodioxolyl (e.g. 1,3-benzodioxoyl, etc.); unsaturated 3 to 8 membered rings containing an oxygen atom and 1 to 2 sulfur atoms such as, but not limited to, dihydrooxathiinyl; saturated 3 to 8 membered rings containing 1 to 2 oxygen atoms and 1 to 2 sulfur atoms such as 1,4-oxathiane; unsaturated condensed rings containing 1 to 2 sulfur atoms such as benzothienyl, benzodithiinyl; and unsaturated condensed heterocyclic rings containing an oxygen atom and 1 to 2 oxygen atoms such as benzoxathiinyl. Heterocyclyl group also include those described above in which one or more S atoms in the ring is double-bonded to one or two oxygen atoms (sulfoxides and sulfones). For example, heterocyclyl groups include tetrahydrothiophene, tetrahydrothiophene oxide, and tetrahydrothiophene 1,1-dioxide. Preferred heterocyclyl groups contain 5 or 6 ring members. More preferred heterocyclyl groups include morpholine, piperazine, piperidine, pyrrolidine, imidazole, pyrazole, 1,2,3-triazole, 1,2,4-triazole, tetrazole, thiomorpholine, thiomorpholine in which the S atom of the thiomorpholine is bonded to one or more O atoms, pyrrole, homopiperazine, oxazolidin-2-one, pyrrolidin-2-one, oxazole, quinuclidine, thiazole, isoxazole, furan, and tetrahydrofuran.

"Substituted heterocyclyl" refers to an unsubstituted heterocyclyl group as defined above in which one of the ring members is bonded to a non-hydrogen atom such as described above with respect to substituted alkyl groups and substituted aryl groups. Examples, include, but are not limited to, 2-methylbenzimidazolyl, 5-methylbenzimidazolyl, 5-chlorobenzthiazolyl, 1-methyl piperazinyl, and 2-chloropyridyl among others.

"Heterocyclylalkyl" refers to unsubstituted alkyl groups as defined above in which a hydrogen or carbon bond of the unsubstituted alkyl group is replaced with a bond to a heterocyclyl group as defined above. For example, methyl ($-CH_3$) is an unsubstituted alkyl group. If a hydrogen atom of the methyl group is replaced by a bond to a heterocyclyl group, such as if the carbon of the methyl was bonded to carbon 2 of pyridine (one of the carbons bonded to the N of the pyridine) or carbons 3 or 4 of the pyridine, then the compound is an unsubstituted heterocyclylalkyl group.

"Substituted heterocyclylalkyl" has the same meaning with respect to unsubstituted heterocyclylalkyl groups that substituted aralkyl groups had with respect to unsubstituted aralkyl groups. However, a substituted heterocyclylalkyl group also includes groups in which a non-hydrogen atom is bonded to a heteroatom in the heterocyclyl group of the heterocyclylalkyl group such as, but not limited to, a nitrogen atom in the piperidine ring of a piperidinylalkyl group.

In one particular embodiment, the optionally substituted aromatic residue may be selected from the group consisting of aromatic and heteroaromatic residues comprising or consisting of phenyl, naphthyl, fluorenyl, pyridine, furane, thiophene, indol, isoindole, quinoline, isoquinoline, oxazole, pyramidine and purine.

The substituted aromatic residues may also be substituted with one or more aliphatic groups, or substituted with one or more heteroatoms, or substituted with one or more aromatic groups, or substituted with one or more heteroaromatic groups.

The at least one hydrophobic functional group may also comprise or consists of an optionally substituted heteroaromatic residue, e.g. a heteroaromatic residue selected from the group consisting of heteroaromatic residues comprising or consisting of furane, pyrrole and thiophene, or a heteroaromatic residue selected from the group consisting of heteroaromatic residues comprising or consisting of fused heteroaromatic compounds, such as a fused heteroaromatic compound selected from the group consisting of indole, benzothiophene, benzotriazene and quinoline.

In the above embodiments, the aromatic residue may be substituted with alkyl or aryl or heteroaryl.

Also, the aromatic residue, or the alkyl or the aryl or the heteroaryl may be substituted with a heteroatom selected from O, N, S and halogen, and/or the aromatic residue, or the alkyl or the aryl or the heteroaryl may be substituted with one or more groups selected from hydroxyl, amino, thiol, halogen, carbonyl, carboxylic acid, ether and ester.

In one embodiment, the ligand comprises at least one amino acid residue comprising a hydrophobic group, e.g. a hydrophobic group which comprises or consists of an aromatic group, or a hydrophobic group which comprises or consists of an aliphatic group.

## Cationic functional groups

Cationic functional groups are positively charged either due to a permanent positive charge or due to an association with an H ion at under normal physiological conditions. Cationic functional groups are attracted by aqueous solution so as to seek the surface positions in the conformation of a ligand when the ligand is in an aqueous medium under normal physiological conditions. Cationic functional groups will seek towards anionic groups of ligand binding partners when the ligand is associated with a binding partner under normal physiological conditions.

The cationic functional group is preferably selected from cationic groups comprising one or more positively charged nitrogen(s), one or more phosphorous atom(s) and/or one or more sulphur atom(s).

Preferred cationic groups comprise a permanent, positively charged nitrogen from groups such as e.g. alkyl ammonium, such as trimethyl ammonium, or triethylammonium, or dimethylammonium, or benzyldimethylammonium, or guanidinium, or a positively charged nitrogen from positively charged heterocycles, such as imidazolinium, piperidinium and pyrrolidinium. Guanidinium is particularly preferred.

Other preferred cationic groups are mono- and disubstituted amines, such as monoalkyl amines, dialkyl amines, heterocyclic amines and aromatic amines which have a partial positive charged in aqueous solutions with pH in the range of 3-8, in particular in the range of 3-7.

The positively charged nitrogen can also be donated by an amino acid residue, such as lysine, arginine, histidine, ornithine, citrulline, diaminobutyric acid, diaminopropionic acid, diaminopentanoic acid, diaminohexanoic acid, diaminopimelic acid, homoarginine, homocitrulline, p-aminophenylalanine and 3-aminotyrosine. Arginine is particularly preferred.

The side chain of an amino acid can provide both one or more hydrophobic functional group(s) and one or more cationic functional group(s). Normally, when amino acids are providing both of said functional groups, at least one hydrophobic functional group and at least one cationic functional group are provided by different amino acids of the ligand. Apart from amino acids, the ligand can comprise other ligand residues as described herein below in more detail.

The term "amino acid" within the scope of the present invention is used in its broadest sense and is meant to include naturally-occurring L-amino acids or residues thereof. The commonly used one- and three-letter abbreviations for naturally-occurring amino acids are used herein (Lehninger, Biochemistry, 2d ed., pp. 71-92, (Worth Publishers: New York, 1975). The term also includes D-amino acids (and residues thereof) as well as chemically-modified amino acids, such as amino acid analogues, including naturally-occurring amino acids that are not usually incorporated into proteins, such as norleucine, as well as chemically-synthesized compounds having properties known in the art to be characteristic of an amino acid.

For example, analogues or mimetics of phenylalanine or proline, which allow the same conformational restriction of a ligand as natural Phe or Pro, are included within the definition of amino acid. Such analogues and mimetics are referred to herein as "functional equivalents" of an amino acid. Other examples of amino acids are listed by Roberts and Vellaccio, The Peptides: Analysis, Synthesis, Biology, Eds. Gross and Meiehofer, Vol. 5, p. 341 (Academic Press, Inc.: N.Y. 1983).

The amino acids are typically linked by amide bonds, but other bonds such as e.g. any one or more bonds selected from -NHN(R)CO-; -NHC(R)CO-; -NHC(RR')CO-; -NHC(=CHR)CO-; $-NHC_6H_4CO-$; $-NHCH_2$ CHRCO-; $-NHCHRCH_2CO-$; $-COCH_2-$; -COS-; -CONR-; -COO-; -CSNH-; $-CH_2NH-$; $-CH_2CH_2-$; $-CH_2S-$; $-CH_2SO-$; $-CH_2SO_2-$; -CH $(CH_3)S-$; -CH=CH-; -NHCO-; -NHCONH-; -CONHO-; $-C(=CH_2)CH_2-$; $-PO_2-NH-$; $-PO_2^-CH_2-$; $-PO_2-CH_2N^+-$; $-SO_2NH-$ can also link amino acid residues of the ligands according to the invention. R (and R') denotes a functional group, such as e.g. a hydrophobic functional group or a cationic group, or another structural entity linking said aforementioned groups. The currently most preferred bond between "amino acids" is the amide bond.

Examples of amino acids that are generally capable of being incorporated into ligands according to the present invention are listed herein below:

Glycyl; aminopolycarboxylic acids, e.g., aspartic acid, p-hydroxyaspartic acid, glutamic acid, β-hydroxyglutamic acid, β-methylaspartic acid, β-methylglutamic acid, β,β-dimethylaspartic acid, γ-hydroxyglutamic acid, β,γ-dihydroxyglutamic acid, β-phenylglutamic acid, Y-methyleneglutamic acid, 3-aminoadipic acid, 2-aminopimelic acid, 2-aminosuberic acid and 2-aminosebacic acid residues; amino acid amides such as glutaminyl and asparaginyl; polyamino- or polybasic-monocarboxylic acids such as arginine, lysine, β-aminoalanine, Y-aminobutyrine, ornithine, citruline, homoarginine, homocitrulline, 5-hydroxy-2,6-diaminohexanoic acid (commonly, hydroxylysine, including allohydroxylysine) and diaminobutyric acid residues; other basic amino acid residues such as histidinyl; diaminodicarboxylic acids such as α,α'-diaminosuccinic acid, α,α'-diaminoglutaric acid, α,α'-diaminoadipic acid, α,α'-diaminopimelic acid, α,α'-diamino-β-hydroxypimelic acid, α,α'-diaminosuberic acid, α,α'-diaminoazelaic acid, and α,α'-diaminosebacic acid residues; imino acids such as proline, 4- or 3-hydroxy-2-pyrrolidine- carboxylic acid (commonly, hydroxyproline, including allohydroxyproline), γ-methylproline, pipecolic acid, 5-hydroxypipecolic acid, $-N([CH_2]_nCOOR_{PR})_2$, wherein n is 1, 2, 3, 4, 5 or 6 and $R_{PR}$ is -H or a protecting group, and azetidine-2-carboxylic acid residues; a mono- or di-alkyl (typically $C_1$-$C_{25}$ branched or normal) amino acid such as alanine, valine, leucine, allylglycine, butyrine, norvaline, norleucine, heptyline, α-methylserine, α-amino-α-methyl-y-hydroxyvaleric acid, α-amino-α-methyl-6-hydroxyvaleric acid, α-amino-α-methyl-ε-hydroxycaproic acid, isovaline, α-methylglutamic acid, α-aminoisobutyric acid, α-aminodiethylacetic acid, α-aminodiisopropylacetic acid, α-aminodi-n-propylacetic acid, α-aminod iisobutylacetic acid, α-aminodi-n-butylacetic acid, α-aminoethylisopropylacetic acid, α-amino-n-propylacetic acid, α-aminodiisoamyacetic acid, α-methylaspartic acid, α-methylglutamic acid, 1-aminocyclopropane-1-carboxylic acid; isoleucine, alloisoleucine, tert-leucine, β-methyltryptophan and α-amino-a-ethyl-β-phenylpropionic acid residues; β-phenylserinyl; aliphatic α-amino-β-hydroxy acids such as serine, β-hydroxyleucine, β-hydroxynorleucine, β-hydroxynorvaline, and α-amino-α-hydroxystearic acid residues; α-Amino, α-, γ-, δ- or ε-hydroxy acids such as homoserine, γ-hydroxynorvaline, δ-hydroxynorvaline and ε-hydroxynorleucine residues; canavinyl and canalinyl; γ-hydroxyornithinyl; 2-Hexosaminic acids such as D-glucosaminic acid or D-galactosaminic acid residues; α-amino-β-thiols, such as penicillamine, β-thiolnorvaline or β-thiolbutyrine residues; other sulfur containing amino acid residues including cysteine; homocystine; β-phenylmethionine; methionine; S-allyl-(L)-cysteine sulfoxide; 2-thiolhistidine; cystathionine; and thiol ethers of cysteine or homocysteine; phenylalanine, tryptophan and ring-substituted amino acids such as the phenyl- or cyclohexylamino acids, α-aminophenylacetic acid, α-aminocyclohexylacetic acid

and α-amino-β-cyclohexylpropionic acid; phenylalanine analogues and derivatives comprising aryl, lower alkyl ($C_1$-$C_6$), hydroxy, guanidino, oxyalkylether, nitro, sulfur or halo-substituted phenyl (e.g., tyrosine, methyltyrosine and o-chloro-, p-chloro-, 3,4-dicloro, o-, m- or p-methyl-, 2,4,6-trimethyl-, 2-ethoxy-5-nitro, 2-hydroxy-5-nitro and p-nitro-phenylalanine); furyl-, thienyl-, pyridyl-, pyrimidinyl-, purine or naphthylalanines; and tryptophan analogues and derivatives including kynurenine, 3-hydroxykynurenine, 2-hydroxytryptophan and 4-carboxytryptophan residues; α-amino substituted amino acid residues including sarcosine (N-methylglycine), N-benzylglycine, N-methylalanine, N-benzylalanine, N-methylphenylalanine, N-benzylphenylalanine, N-methylvaline and N-benzylvaline; and α-Hydroxy and substituted α-hydroxy amino acid residues including serine, threonine, allothreonine, phosphoserine and phosphothreonine residues. Also of interest are hydrophobic amino acids such as mono-or dialkyl or aryl amino acids, cycloalkylamino acids, and the like.

*Preferred ligands*

Selected ligand residues as cited herein below are denoted:

PPC: 4-Phenyl piperidine-4-carboxylic acid
Dap: Diaminopropionic acid
L-Orn: L-Ornithine
DPPAA: 2,4-Di-tert-pentylphenoxy acetic acid
DMBA: 3,5-Dimethoxybenzoic acid
TMPPA: 3-(3,4,5-Trimethoxyphenyl)propionic acid
DBHPA: 3-(3,5-Di-tert-butyl-4-hydroxyphenyl)propionic acid
1H2NA: 1-Hydroxy-2-naphthoic acid
DPPA: 3,3-Diphenylpropionic acid
SAA: Salicylic acid
DBBA: 3,5-Di-tert-butylbenzoic acid
DPPBA: 4-(2,4-Di-tert-pentylphenoxy)butyric acid
TEBA: 3,4,5-Triethoxybenzoic acid
PCAA: α-Phenyl cyclopentane acetic acid
MDCA: 3,4-(Methylenedioxy) cinnamic acid
Gly: Glycine
L-Phe: L-Phenylalanine
L-Arg: L-Arginine
L-His: L-Histidine
L-Trp: L-Tryptophan
L-Pro: L-Proline
L-Asn: L-Asparagine
L-Lys: L-Lysine
L-Asp: L-Aspartic acid
D-Phe: D-Phenylalanine
D-Arg: D-Arginine
D-Tyr: D-Tyrosine
D-Ser: D-Serine
D-Trp: D-Tryptophan
D-Pro: D-Proline
D-Leu: D-Leucine
Ahx: 6-Aminohexanoic acid
Aib: α-Aminoisobutyric acid
DBHBA: 3,5-Di-tert-butyl-4-hydroxybenzoic acid
INA: Isonipectoic acid
Nle: L-Norleucine
PPC: 4-Phenyl-piperidine 4-carboxylic acid
SAA: Salicylic acid
3HBA: 3-Hydroxybenzoic acid
4HBA: 4-Hydroxybenzoic acid

In the main class of preferred affinity ligands, each ligand comprises one or more hydrophobic functional group (s) and one or more cationic functional group(s), wherein at least one hydrophobic functional group is separated from at least one cationic functional group by a through bond distance of from 5 Å, to 20 Å, and wherein said ligand has a

molecular weight of from 120 Da to 1,500 Da.

In one preferred embodiment, the affinity ligand comprising or consisting of covalently linked residues $X_1$-$X_2$-$X_3$, wherein optionally $X_1$, $X_2$ and/or $X_3$, in particular $X_1$ and/or $X_3$, is associated with a linker residue, L.

Preferably $X_1$, $X_2$, $X_3$, and the optional linker residue are preferably highly stable molecules, which can withstand repeated exposure to harsh chemical conditions (e.g. 1 M strong acid or 1 M strong base) and biological conditions (e.g. high protease activity). Likewise, the respective bonds between each of $X_1$, $X_2$, $X_3$, and the optional linker residue are highly stable and can withstand harsh chemical and biological conditions.

The residue $X_1$ is preferably selected from the group consisting of Arg, Phe, PPC, DBHBA, SAA, DAP, DAB, $(DBHBA)_2$-DAP, $(MDCA)_2$-DAP, DPBBA, DBBA, PCAA, DPPAA, Trp, TMPPA, DBHPA, wherein each member is optionally further selected from an isolated and optically active residue and a racemic mixture comprising both of the optically active isomers of the same residue. In one preferred variant, $X_1$ is selected from the group consisting of L-Arg, D-Lys, D-Phe, D-Pro, INA, PPC, DBHBA, 3HBA, 4HBA and SAA.

The residue $X_2$ is preferably selected from the group consisting of Arg, Asn, Leu, Lys, Phe, Pro, PPC, DAP, DAB, His, Trp, Tyr, Ser, wherein each member is optionally further selected from an isolated and optically active residue and a racemic mixture comprising both of the optically active isomers of the same residue. In one preferred variant, $X_2$ is selected from the group consisting of L-Arg, L-Asn, D-Leu, D-Lys, D-Phe, D-Pro, L-Pro, AIB, AHX, INA, Nle and PPC.

The residue $X_3$ is preferably selected from the group consisting of Arg, Asn, Pro, PPC, Asp, Orn, (1H2NA)Dap wherein each member is optionally further selected from an isolated and optically active residue and a racemic mixture comprising both of the optically active isomers of the same residue. In particular, $X_3$ is preferably selected from the group consisting of L-Arg, L-Asn, D-Lys, D-Phe, D-Pro, L-Pro and PPC.

A currently promising set of ligands having affinity for an antibody, such as a monoclonal antibody, are the ligands comprising or consisting of one or more of the sets (corresponding to $X_1$-$X_2$-$X_3$):

D-Phe-(L)Arg-(L)Arg
L-Arg-D-Phe-(L)Arg
PPC-D-Pro-(L)Arg
PPC-D-Leu-PPC
INA-D-Phe-PPC
PPC-Aib-PPC
D-Phe-(L)Arg-(L)Arg
L-Arg-D-Phe-(L)Arg
L-Arg-(L)Arg-D-Phe
PPC-(L)Arg-D-Pro
D-Pro-PPC-(L)Arg
L-Arg-D-Pro-PPC
L-Arg-D-Lys-(L)Arg
L-Arg-(L)Arg-D-Lys
D-Lys-INA-(L)Arg
INA-(L)Arg-D-Lys
PPC-Ahx-PPC
PPC-Nle-PPC
SAA-(L)Arg-(L)Pro
SAA-(L)Pro-(L)Arg
DBHBA-(L)Arg-(L)Asn
DBHBA-(L)Asn-(L)Arg
3HBA-(L)Arg-(L)Pro
3HBA-(L)Pro-(L)Arg
4HBA-(L)Arg-(L)Pro
4H BA-(L) Pro-(L)Arg

In another currently promising embodiment, the ligand comprises or consists of less than 5, such as less than 4 residues, for example 3 residues selected from the group consisting of D-Leu; D-Lys; D-Phe; D-Pro; L-Arg; L-Asn; L-Pro; Ahx; Aib; DBHBA; INA; Nle; PPC; SAA; 3HBA and 4HBA. The ligand can thus consist of 3 residues, $X_1$-$X_2$-$X_3$, individually selected from the group consisting of D-Leu; D-Lys; D-Phe; D-Pro; L-Arg; L-Asn; L-Pro; Ahx; Aib; DBHBA; INA; Nle; PPC; SAA; 3HBA and 4HBA.

When the ligand comprises or consists of 3 residues, $X_1$-$X_2$-$X_3$,

$X_1$ is preferably selected from the group consisting of D-Lys; D-Phe; D-Pro; L-Arg; DBHBA; INA; PPC; SAA; 3HBA

and 4HBA,

$X_2$ is preferably selected from the group consisting of D-Leu; D-Lys; D-Phe; D-Pro; L-Arg; L-Asn; L-Pro; Ahx; AIB; INA; Nle; PPC; and

$X_3$ is preferably selected from the group consisting of D-Lys; D-Phe; D-Pro; L-Arg; L-Asn; L-Pro; PPC.

In a further embodiment, the ligand comprises or consists of 3 covalently linked residues, $X_1$-$X_2$-$X_3$, wherein said covalently linked residues are further covalently linked to the linker residue L of the entity L-PM, wherein PM is the solid support material, preferably a polymer matrix optionally in cross-linked and/or beaded form,
wherein $X_1$ is a natural or non-natural amino acid in D- and/or L-configuration, or a carboxylic acid residue comprising an optionally substituted aromatic group,
wherein $X_2$ is a natural or non-natural amino acid in either D- and/or L-configuration, or a carboxylic acid residue comprising an optionally substituted aromatic group, with the proviso that $X_2$ is not a threonine residue, and
wherein $X_3$ is a natural or non-natural amino acid in either D- and/or L-configuration, or a carboxylic acid residue comprising an optionally substituted aromatic group,
wherein at least one of $X_1$, $X_2$ and $X_3$ comprises a cationic functional group, and
wherein at least one of $X_1$, $X_2$ and $X_3$ comprises a hydrophobic functional group.
wherein $X_1$ is selected from L-Arg, D-Lys, D-Phe, D-Pro, INA, PPC, DBHBA, 3HBA, 4HBA and SAA;
wherein $X_2$ is selected from L-Arg, L-Asn, D-Leu, D-Lys, D-Phe, D-Pro, L-Pro, AIB, AHX, INA, NLE and PPC; and
wherein $X_3$ is selected from L-Arg, L-Asn, D-Lys, D-Phe, D-Pro, L-Pro and PPC.

In a still further promising embodiment, the ligand comprising or consisting of 3 covalently linked residues, $X_1$-$X_2$-$X_3$,
wherein said covalently linked residues are covalently linked to a linker L of the entity L-PM,
wherein $X_1$ is a natural or non-natural amino acid in D- and/or L-configuration, or a carboxylic acid residue comprising an optionally substituted aromatic group,
wherein $X_2$ is a natural or non-natural amino acid in either D- and/or L-configuration, or a carboxylic acid residue comprising an optionally substituted aromatic group, with the proviso that $X_2$ is not a threonine residue, and
wherein $X_3$ is a natural or non-natural amino acid in either D- and/or L-configuration, or a carboxylic acid residue comprising an optionally substituted aromatic group,
wherein at least one of $X_1$, $X_2$ and $X_3$ comprises a cationic functional group, and
wherein at least one of $X_1$, $X_2$ and $X_3$ comprises a hydrophobic functional group.
wherein $X_1$ is selected from L-Arg, D-Lys, D-Phe, D-Pro, INA, PPC, DBHBA, 3HBA, 4HBA and SAA;
wherein $X_2$ is selected from L-Arg, L-Asn, D-Leu, D-Lys, D-Phe, D-Pro, L-Pro, AIB, AHX, INA, NLE and PPC; and
wherein $X_3$ is selected from L-Arg, L-Asn, D-Lys, D-Phe, D-Pro, L-Pro and PPC.

In one embodiment, the linker residue L attached to the polymer matrix is cleavable by acids, bases, temperature, light, or by contact with a chemical reagent. As before, the linker attached to the polymer matrix may be one selected from (3-formylindol-1-yl)acetic acid, 2,4-Dimethoxy-4'-hydroxy-benzophenone, HMPA, HMPB, HMPPA, Rink acid, Rink amide, Knorr linker, PAL linker, DCHD linker, Wang linker and Trityl linker. Ligands bound to a polymer matrix via cleavable linker can be used for analytical purposes, e.g. for diagnostic applications.

In one embodiment of the present invention, $X_1$, $X_2$, and $X_3$ are chosen from the group of natural amino acids and their stereoisomers.

It has been found that the inclusion of at least one amino acid comprising a side chain guanidino or amino group, in particular guanidino groups, provide typically provide advantageous properties with respect to binding capacity. Hence, particularly relevant amino acids to include in the ligands are arginine, homoarginine, lysine, homolysine and ornitine, in particular arginine and homoarginine.

A particularly promising subclass of ligands is the one where the ligands comprise two arginine moieties. Hence, particularly favoured ligands within this embodiment are,

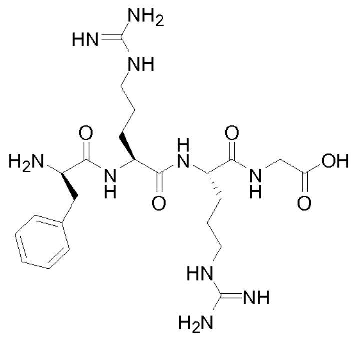

$H_2N$-(D)Phe-(L)Arg-(L)Arg-Gly-OH
and

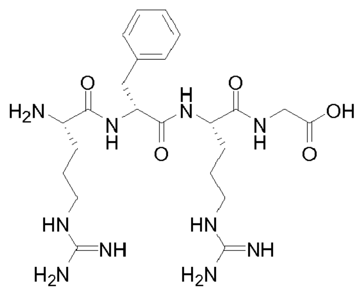

$H_2N$-(L)Arg-(D)Phe-(L)Arg-Gly-OH.

In a further embodiment $X_1$, $X_2$, and $X_3$ are chosen from the group of natural and unnatural amino acids.

Hence, for a still further promising subclass of ligands,

$X_1$ is chosen from the group consisting of Arg, Phe, PPC, DBHBA, SAA, DAP, DAB, $(DBHBA)_2$-DAP, $(MDCA)_2$-DAP, DPBBA, DBBA, PCAA, DPPAA, Trp, TMPPA, and DBHPA,

$X_2$ is chosen from the group consisting of Arg, Asn, Leu, Lys, Phe, Pro, PPC, DAP, DAB, His, Trp, Tyr, and Ser,

$X_3$ is chosen from the group consisting of Arg, Asn, Pro, PPC, Asp, Orn, and (1H2NA)Dap.

Particularly favoured ligands within this subclass are:

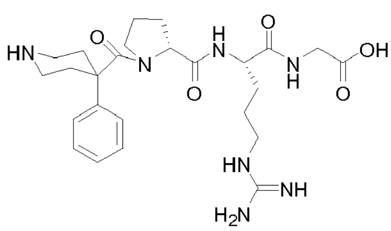

HN-PPC-(D)Pro-(L)Arg-Gly-OH
and

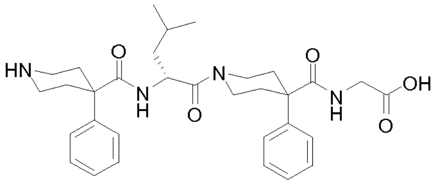

HN-PPC-(D)Leu-PPC-Gly-OH

In a still further subclass of ligands, each ligand comprises one or more substituted or unsubstituted phenyl or naphtyl groups and one or more primary amine or guanidine.

Particularly favoured ligands within this embodiment are,

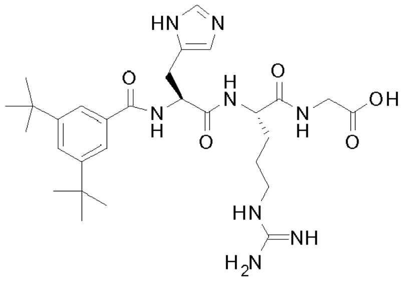

DBBA-(L)His-(L)Arg-Gly-OH,
DBBA-His-Arg-Gly-OH,
DBBA-His-Arg-Arg-Gly-OH

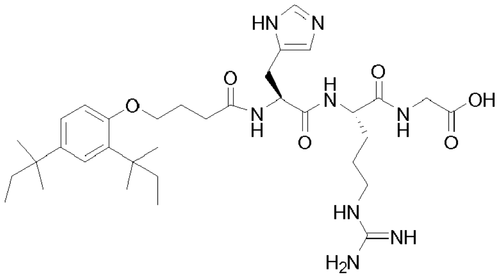

DPPBA-(L)Phe-(L)Arg-Gly-OH,

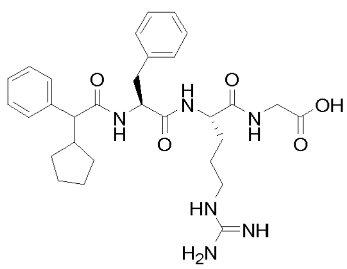

PCAA-(L)Phe-(L)Arg-Gly-OH,

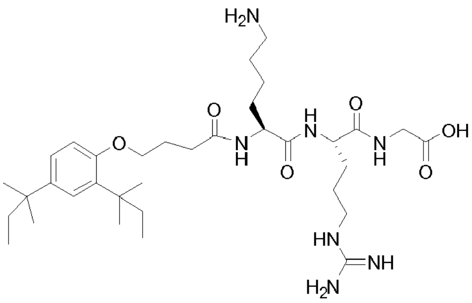

DPPBA-(L)Lys-(L)Arg-Gly-OH

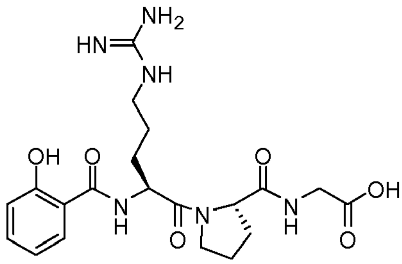

SAA-(L)Arg-(L)Pro-Gly-OH

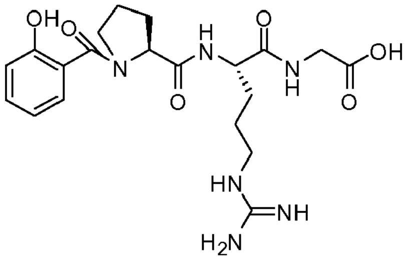

SAA-(L)Pro-(L)Arg-Gly-OH

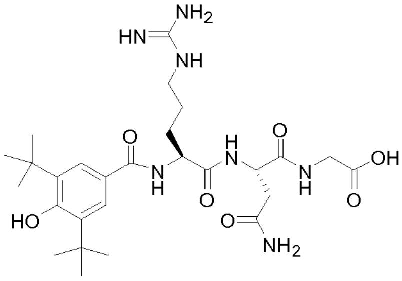

DBHBA-(L)Arg-(L)Asn-Gly-OH

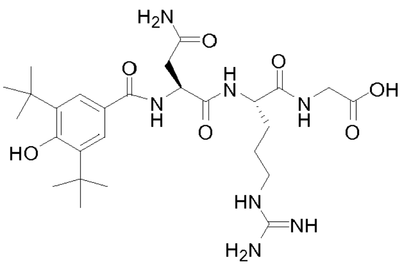

DBHBA-(L)Asn-(L)Arg-Gly-OH

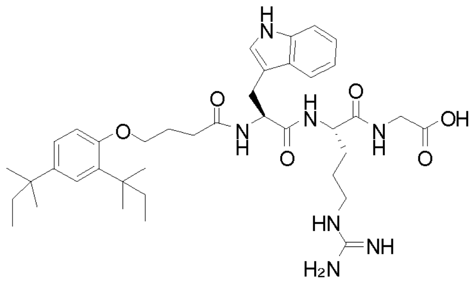

DPPBA-(L)Trp-(L)Arg-Gly-OH

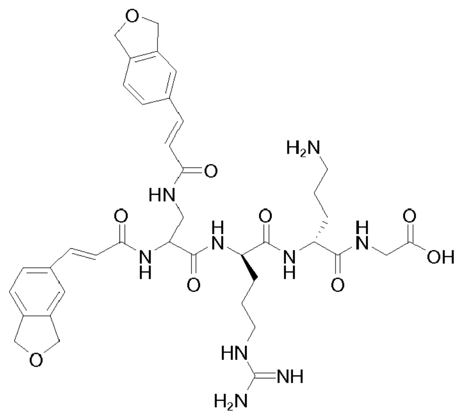

$(MDCA)_2$-DAP-(L)Arg-(L)Orn-Gly-OH

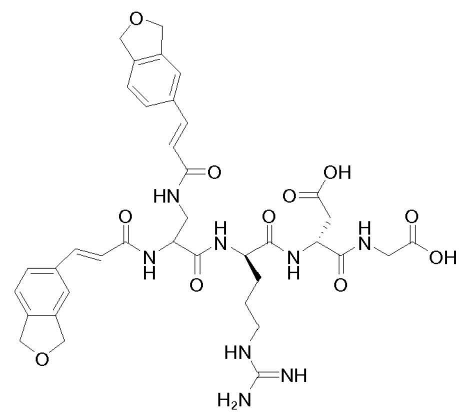

$(MDCA)_2$-DAP-(L)Arg-(L)Asp-Gly-OH

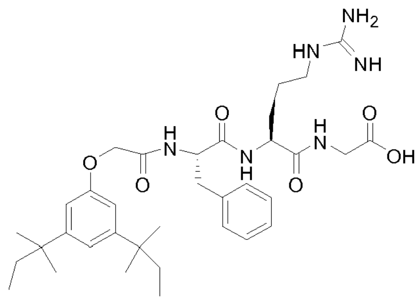

DPPAA-(L)Phe-(L)Arg-Gly-OH

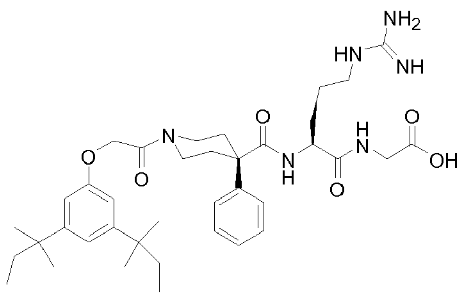

DPPAA-PPC-(L)Arg-Gly-OH

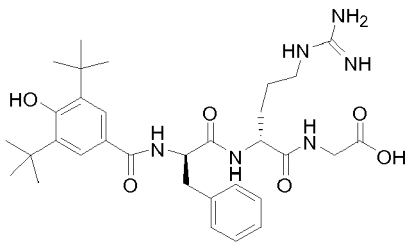

DBHBA-(D)Phe-(D)Arg-Gly-OH

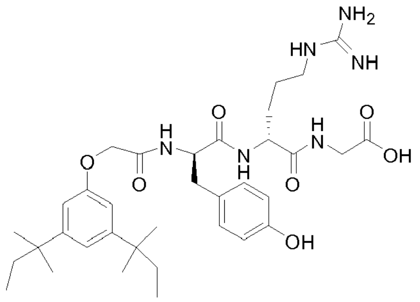

DPPAA-(D)tyr-(D)Arg-Gly-OH

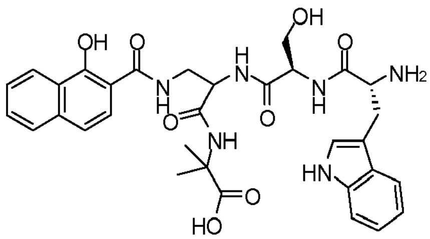

$H_2N$-(D)Trp-(D)Ser-(1H2NA)DAP-Aib-OH,

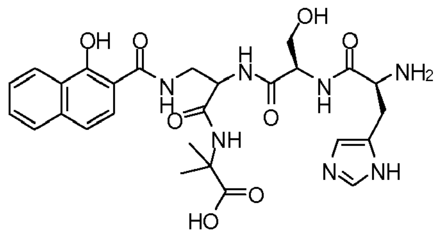

$H_2N$-(L)His-(D)Ser-(1H2NA)DAP-Aib-OH,

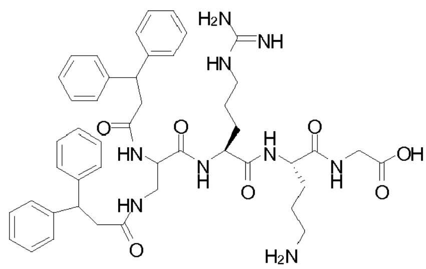

$(DPPA)_2$-DAP-(L)Arg-(L)Orn-Gly-OH

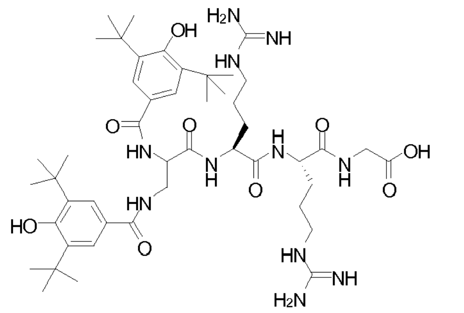

(DBHBA)$_2$-DAP-(L)Arg-(L)Arg-Gly-OH,
DBHBA-DAP-Arg-Gly-OH,
DBHBA-DAP-Arg-Arg-Gly-OH,
(DBHBA)$_2$-DAP-Arg-Gly-OH,
(DBHBA)$_3$-DAP-Arg-Arg-Gly-OH,
(DBHBA)$_3$-DAP-Arg-Arg-Gly-OH,

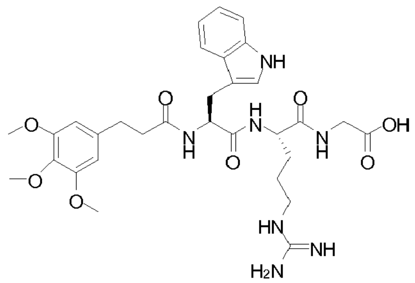

TMPPA-(L)Trp-(L)Arg-Gly-OH

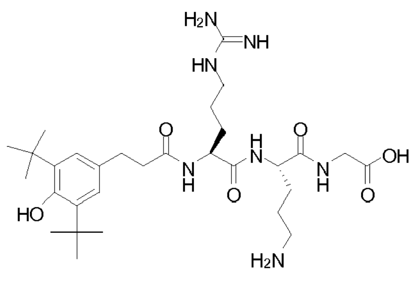

DBHPA-(L)Arg-(L)Orn-Gly-OH

*Alternative affinity ligands*

Another currently promising class of affinity ligands is the one where the ligands contain one or more hydrophobic groups and one or more heteroaromatic groups.

Hence, in another main class of preferred affinity ligands, each ligand comprises one or more hydrophobic functional group(s) and one or more heteroaromatic functional group(s),
wherein at least one hydrophobic functional group is separated from at least one heteroaromatic functional group by a through bond distance of from 5 Å, to 20 Å, and wherein said ligand has a molecular weight of from 120 Da to 5,000 Da.

Preferably, the affinity resin having such an affinity ligand immobilised thereon has a binding capacity larger than 5 mg monoclonal antibody per mL of affinity resin.

The term "heteroaromatic functional group(s)" is intended to encompass the heteroaromatic species defined under "Hydrophobic functional groups comprising aromatic residues" and "Cationic functional groups" hereinabove.

The specifications and preferences with respect to through bond distances, through space distances, molecular

weight, etc. given further above for the main class of affinity ligands also apply for this class of affinity ligands, although it should be understood that "distances" referred to are between the hydrophobic functional group(s) and heteroaromatic functional group(s).

An example of a ligand within this class is

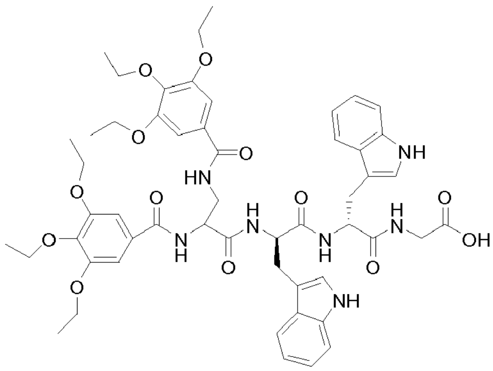

$(TEBA)_2$-DAP-(L)Trp-(L)Trp-Gly-OH.

*Solid support material*

The ligands according to the invention are covalently immobilised to a solid support material, such as a polymer resin, a surface of a polymeric material, a glass surface, etc, and can be used for purifying and/or isolating antibodies, such as monoclonal antibodies.

The solid support material typically comprises a polymer matrix, e.g. a cross-linked polymer matrix. Most often, the polymer matrix is beaded, alternatively, the polymer matrix is prepared as a monolithic unit. Preferably, at least the surface of the beaded polymer matrix comprises hydrophilic moieties.

Hence, the solid support material is typically in the form of a resin, a monolith, a filter, a plate, such as a micro-array, a fibre, a sensor, such as a cantelever, a surface plasmon resonance sensor, or a quartz crystal microbalance.

A resin is a porous solid material used for separation of compounds in a fluid. Resins can be cross-linked polymers, ceramic materials, such as inorganic oxides, e.g. aluminium oxide, silicium oxide. Resins are provided as particles, such as spherical particles or irregular particles.

The ligands are characterised by a binding capacity which is preferably larger than 5 mg of antibody per mL of wet resin, such as larger than 6 mg of antibody per mL of resin, for example larger than 7 mg of antibody per mL of resin, such as larger than 8 mg of antibody per mL of resin, for example larger than 9 mg of antibody per mL of resin, such as larger than 10 mg of antibody per mL of resin, for example larger than 12 mg of antibody per mL of resin, such as larger than 14 mg of antibody per mL of resin, for example larger than 16 mg of antibody per mL of resin, such as larger than 18 mg of antibody per mL of resin, for example larger than 20 mg of antibody per mL of resin, such as larger than 22 mg of antibody per mL of resin, for example larger than 24 mg of antibody per mL of resin, such as larger than 26 mg of antibody per mL of resin, for example larger than 28 mg of antibody per mL of resin, such as larger than 30 mg of antibody per mL of resin, for example larger than 35 mg of antibody per mL of resin, such as larger than 40 mg of antibody per mL of resin, for example larger than 45 mg of antibody per mL of resin, such as larger than 50 mg of antibody per mL of resin, such as e.g. at the most 500 mg of antibody per mL of resin.

The expression "binding capacity" denotes the capacity of the resin to bind a target compound, such as e.g. an antibody. The binding capacity is given in mg target (e.g. antibody) per mL of resin (or other form of beaded material), and can be measured by passing a solution of the pure target with a known concentration through the resin and measuring the volume until breakthrough of the target compound. The binding capacity is then calculated as the breakthrough volume multiplied by the concentration and divided by the bed volume. As used herein, binding capacity denotes the capacity of the resin to bind an antibody. The binding capacity is given in mg antibody per milliliter of resin, and can be measured by passing an aqueous buffered solution (50 mM Na-Phosphate, pH=7.0) of the pure antibody with a known concentration and recording the development of the UV absorbance (280 nm) of the solution exiting the resin. The breakthrough volume is the volume of solution that has exited from the resin at the point where the UV absorbance has reached 5% of its terminal value. The binding capacity is then calculated as the breakthrough volume multiplied by the concentration of antibody in the feed solution and divided by the bed volume.

When the ligand is attached to the surface of an essentially non-porous material, such as e.g. and array surface or a sensor surface, the binding capacity of the ligand is measured as mass of protein bound per surface area. The

ligands are characterised by a binding capacity which is preferably larger than 1 ng of antibody per $cm^2$, such as larger than 5 ng of antibody per $cm^2$, for example larger than 10 ng of antibody per $cm^2$, such as larger than 50 ng of antibody per $cm^2$, for example larger than 100 ng of antibody per $cm^2$.

Suitable examples of resins are disclosed herein below in more detail.

Polymer matrices

The ligand can be associated covalently to a solid support such as a porous, inorganic matrix or a polymer matrix, optionally in cross-linked and/or beaded form or in a monolithic porous entity. Preferably, the pores of the polymer matrix are sufficiently wide for the target protein to diffuse through said pores and interact with the ligand on the inner surface of the pores. For a monoclonal antibody with molar mass approx. 150 kDa an average pore diameter of 50-200 nm is preferred, such as approx. 100 nm. A number of commercially available suitable polymer resins are available, e.g. Sepharose™, Fractogel™, CIMGEL™, Toyopearl.

The beaded and optionally cross-linked polymer matrix in one embodiment comprises a plurality of hydrophilic moieties. The hydrophilic moieties can be polymer chains which, when cross-linked, form the cross-linked polymer matrix. Examples include e.g. polyethylene glycol moieties, polyamine moieties, polyvinylamine moieties, and polyol moieties.

In some embodiments, the core and/or the surface of a beaded polymer matrix comprises a polymeric material selected from the group consisting of polyvinyls, polyacrylates, polyacrylamides, polystyrenes, polyesters and polyamides.

The beaded polymer matrix can also be selected from the group consisting of PS, POEPS, POEPOP, SPOCC, PEGA, CLEAR, Expansin, Polyamide, Jandagel, PS-BDODMA, PS-HDODA, PS-TTEGDA, PS-TEGDA, GDMA-PMMA, PS-TRPGDA, ArgoGel, Argopore resins, ULTRAMINE, crosslinked LUPAMINE, high capacity PEGA, Silica, Fractogel, Sephadex, Sepharose, Glass beads, crosslinked polyacrylates, and derivatives of the aforementioned; in particular, the polymer matrix is selected from the group consisting of SPOCC, PEGA, HYDRA, POEPOP, PEG-polyacrylate copolymers, polyether-polyamine copolymers, and cross-linked polyethylene diamines.

Apart from the above-mentioned examples, any material capable of forming a polymer matrix can in principle be used in the production of beads of the invention. Preferably, the core material of a bead is polymeric. In some embodiments, the core comprises or consists of hydrophilic polymeric material. In other embodiments, the core comprises or consists of hydrophobic polymeric material. In some embodiments, the surface of the beads comprises or consists of the same material as the core.

Resins useful for large-scale applications may be one of the above mentioned or other commercial resins such as Sephadex™, Sepharose™ , Fractogel™, CIMGEL™, Toyopearl, crosslinked agarose, and macroporous polystyrene or polyacrylate. The matrix may also be of a mainly inorganic nature, such as macroporous glass or clay minerals, or combinations of resins and and inorganics, such as Ceramic HyperD™.

Polymer beads according to the invention can be prepared from a variety of polymerisable monomers, including styrenes, acrylates and unsaturated chlorides, esters, acetates, amides and alcohols, including, but not limited to, polystyrene (including high density polystyrene latexes such as brominated polystyrene), polymethylmethacrylate and other polyacrylic acids, polyacrylonitrile, polyacrylamide, polyacrolein, polydimethylsiloxane, polybutadiene, polyisoprene, polyurethane, polyvinylacetate, polyvinylchloride, polyvinylpyridine, polyvinylbenzylchloride, polyvinyltoluene, polyvinylidenechloride and polydivinylbenzene. In other embodiments, the beads are prepared from styrene monomers or PEG based macromonomers. The polymer is in preferred embodiments selected from the group consisting of polyethers, polyvinyls, polyacrylates, polymethacrylates, polyacylamides, polyurethanes, polyacrylamides, polystyrenes, polycarbonates, polyesters, polyamides, and combinations thereof. Highly preferred surface and core moieties include cross-linked PEG moieties, polyamine moieties, polyvinylamine moieties, and polyol moieties.

A preferred hydrophobic polymer to be used for production of beads of the composition of the invention is PS-DVB (polystyrene divinylbenzene). PS-DVB has been widely used for solid-phase peptide synthesis (SPPS), and has more recently demonstrated utility for the polymer-supported preparation of particular organic molecules (Adams et al. (1998) J.Org.Chem. 63:3706-3716). When prepared properly (Grøtli et al. (2000) J.Combi.Chem.2:108-119), PS-DVB supports display excellent properties for chemical synthesis such as high loading, reasonable swelling in organic solvents and physical stability.

In one embodiment of the present invention, the ligand is associated to the surface of a sensor or an array plate and used to detect and/or quantify antibodies in a biological sample.

When used herein, the term "biological sample" includes natural samples or samples obtained from industrial processes, e.g. recombinant processes, and include "body fluid", i.e. any liquid substance extracted, excreted, or secreted from an organism or tissue of an organism. A body fluid need not necessarily contain cells. Body fluids of relevance to the present invention include, but are not limited to, whole blood, serum, urine, plasma, cerebral spinal fluid, tears, milk, sinovial fluid, and amniotic fluid.

In a further embodiment, a plurality of ligands are associated to the surface of an array plate and arranged in a plurality of spots, with each spot representing one ligand. Such a functionalized array can be used to detect the presence of antibodies in a solution. Such an array can be used for diagnostic applications to detect the presence of certain antibodies in a biological sample.

In a further embodiment, a plurality of ligands are associated to the binding surface of a cantilever sensor for detection and optionally quantification of antibodies. A plurality of affinity ligands can be associated to a plurality of cantilevers with each cantilever representing one ligand. Such a functionalized array can be used to detect the presence of various antibodies in a solution. Such a multi-sensor can be used for diagnostic applications to detect the presence of certain antibodies in a biological sample.

Linkers

The above-mentioned ligand is covalently immobilized to a solid support material, possibly through a linker. In preferred embodiments, the ligand is covalently attached to a linker which is covalently attached to the polymer matrix. General techniques for linking of affinity ligands to solid support materials can be found in Hermanson, Krishna Mallia and Smith, Immobilized Affinity Ligand Techniques", Academic Press, 1992.

Linkers are used for linking the ligand to a solid support such as e.g. a polymer matrix or an inorganic support. Preferably, the linker forms a strong and durable bond between the ligand and the solid support. This is particularly important, when the solid support material of the present invention is to be used for repeated purification of monoclonal antibodies or fragments thereof.

However, in one embodiment of the present invention, linkers can be selectively cleavable. This can be useful when the solid support is to be used for analytical purposes.

Amino acids and polypeptides are examples of typical linkers. Other possible linkers include carbohydrates and nucleic acids.

In one embodiment, the linker residue L attached to the polymer matrix is cleavable by acids, bases, temperature, light, or by contact with a chemical reagent. In particular, the linker attached to the polymer matrix can be (3-formylindol-1-yl)acetic acid, 2,4-dimethoxy-4'-hydroxy-benzophenone, HMPA, HMPB, HMPPA, Rink acid, Rink amide, Knorr linker, PAL linker, DCHD linker, Wang linker and Trityl linker.

The ligand can be associated with the solid support through a linker having a length of preferably less than 50 Å, such as a length of from 3 to 30 Å, for example a length of from 3 to 20 Å, such as a length of from 3 to 10 Å.

The linker can be attached to a hydrophobic functional group or to a cationic functional group, or to a structural entity of the ligand joining a hydrophobic functional group and a cationic functional group. In one embodiment, the linker is attached to a cationic functional group. Preferably, however, the linker is attached to the affinity ligand via a carboxylic acid group, or an amino group, in particular via a carboxylic acid group.

The linker may also comprise a plurality of covalently linked subunits, e.g. such that the subunits are selected from identical and non-identical linker subunits. In one variant, the linker is flexible and comprises from 3 to preferably less than 50 identical or non-identical, covalently linked subunits.

In a preferred embodiment of the present invention, the linker L is selected from the group consisting of glycine, alanine, 3-aminopropionic acid, 4-aminobutanoic acid, and HMBA.

The linker can also be selected from the group consisting of polydispersed polyethylene glycol; monodispersed polyethylene glycol, such as triethylene glycol, tetraethylene glycol, pentaethylene glycol, hexaethylene glycol, heptaethylene glycol; an amino acid; a dipeptide; a tripeptide; a tetrapeptide; a pentapeptide; a hexapeptide; a heptapeptide; octapeptide; a nonapeptide; a decapeptide, a polyalanine; a polyglycine, a polylysines, a polyarginine, including any combination thereof.

*Preferred embodiments*

One preferred embodiment relates to a solid support material having covalently immobilized thereon an affinity ligand, said ligand comprising one or more hydrophobic functional group(s) and one or more cationic functional group(s), wherein at least one hydrophobic functional group is separated from at least one cationic functional group by a through bond distance of from 5 Å, to 20 Å,
wherein said ligand has a molecular weight of from 120 Da to 1,500 Da;
wherein the ligand comprises one or more amino acids having side chain guanidino groups and one or more amino acids having side chain substituted phenyl or naphtyl groups.

In view of the above, it has been found that particularly interesting ligands are those selected from the group consisting of DBBA-(L)His-(L)Arg-Gly-OH and $(DBHBA)_2$-DAP-(L)Arg-(L)Arg-Gly-OH.

*A composition*

The present invention also provides a solid support material having covalently immobilized thereon a plurality of different affinity ligands. Such a composition can be used to separate various antibodies from a biological sample.

*Method for the isolation of antibodies*

Another aspect of the present invention relates to a method for the isolation of biomolecules, such as proteins, e.g. antibodies, in particular monoclonal antibodies, the method comprising the steps of (i) providing a solid support material having covalently immobilized thereon an affinity ligand as defined herein, (ii) providing a sample putatively containing an antibody having an affinity for said ligand, (iii) contacting said ligand with said sample putatively containing said antibody, (iv) binding selectively said antibody when said antibody is contained in said sample and (v) isolating selectively said antibody when said antibody is contained in said sample.

In one variant, the method comprises the steps of providing a ligand as defined herein, attaching said ligand to a solid support, such as e.g. a beaded and/or cross-linked polymer matrix, providing a sample putatively containing an antibody having an affinity for said ligand, contacting said ligand with said sample putatively containing said antibody, binding selectively said antibody when said antibody is contained in said sample and isolating selectively said antibody when said antibody is contained in said sample.

Preferably, the binding capacity of the ligand is larger than 5 mg of antibody per mL of resin, or even more as described further above under "Solid Support Material".

It should be understood that the handling of the solid support material and the procedure essentially follows that of conventional affinity chromatographic techniques.

EXAMPLES

The present invention is further illustrated by the following non-limiting examples.

The examples describe how large libraries can be designed and tested for their affinity towards monoclonal antibodies.

*Materials and Methods*

All reagents and solvents obtained from commercial suppliers and used without further purification. All solvents used were of HPLC grade kept over molecular sieves. Fmoc-protected amino acids and HMBA linker were obtained from Bachem AG, Bubendorf, Switzerland. PPC and ACC were purchased from Neosystem, Strasbourg, France. Sepharose™ was purchased from Sterogene and Fractogel™ was purchased from Merck. All the ligands were synthesised on PEGA1900 (Versabeads™ A) resin. A 20 well multiple column peptide synthesizer was used for the combinatorial library synthesis. $^1$H NMR spectra were recorded in $CDCl_3$ solutions with a Varian Unity Inova 500 MHz spectrometer. Chemical shifts are reported in $\delta$ values relative to tetramethylsilane. The ESI-mass spectra were recorded on a Waters Global Ultima mass spectrometer and MALDI-TOF spectra were recorded on a Bruker Reflex III mass spectrometer using $\alpha$-cyano-4-hydroxycinnamic acid as matrix. Analytical and preparative reverse-phase HPLC separations were performed on a Waters HPLC system using analytical Zorbax 300SB-$C_{18}$ (4.5 x 50 mm) and Delta PAK (25 x 300 mm) $C_{18}$ columns with a flow rate of 1 $cm^3min^{-1}$ and 10 $cm^3min^{-1}$, respectively. Detection was at 215 nm on a multiwavelength detector (Waters 490E) for analytical purposes and a photodiode array detector (Waters M991) was used for preparative separations. A solvent system consisting of A: 0.1% TFA in water and B: 0.1% TFA in 90:10 acetonitrile/water was used.

*Labelling of Fc fragment*

The Fc fragment of an antibody was purified by dialysis in bicarbonate buffer (pH 8). The purified Fc fragment was treated with Oregon Green dye (50 x) in DMF and kept for 1 hour at room temperature. The reaction was stopped by adding hydroxylamine (1.5 M, pH 8.5). The labelled polypeptide was purified by dialysis in bicarbonate buffer (pH 8).

*Example 1. Synthesis of ligands*

The solid phase synthesis followed the scheme:

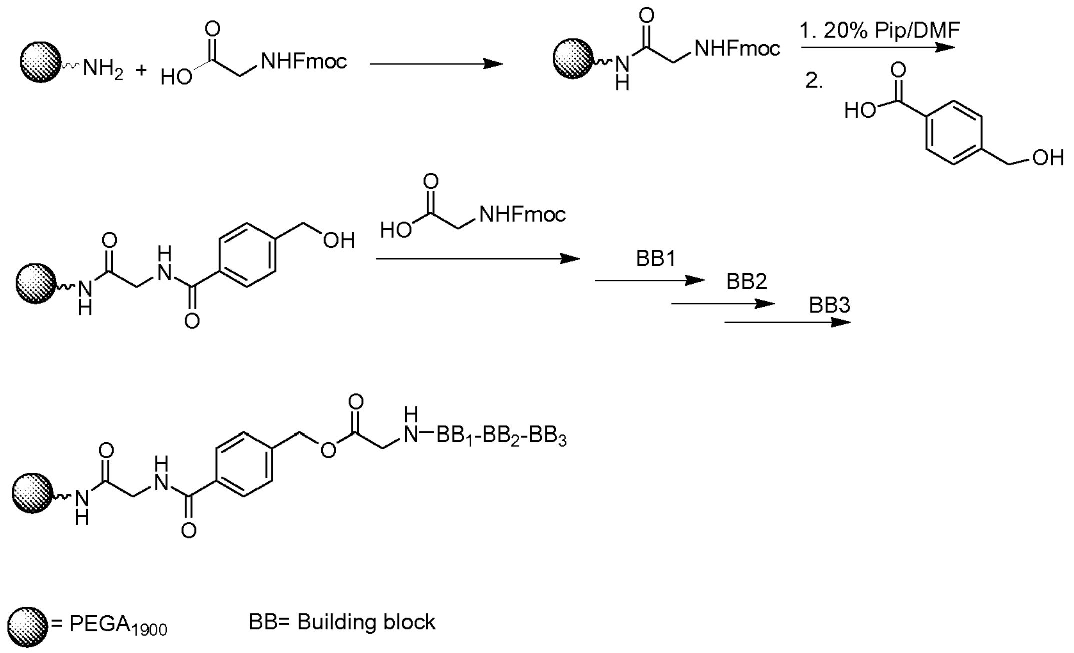


All compounds are synthesized on a polyethyleneglycol-acrylamide based amino functional resin, PEGA1900. The ligands were attached to the glycine derivatised resin via base labile *p*-hydroxymethylbenzoic acid HMBA linker.

The building block (acid/amino acid; 3 equiv) dissolved in DMF and N-ethylmorpholine (4 equiv) and TBTU (2.88 equiv) were added. The reaction mixture was kept for 5 min and added to the swollen resin in DMF for 2h. The resin was washed with DMF, EtOH-DMF and DCM.

<u>Incubation of synthesised compounds in labelled Fc fragment</u>

The beads (10 mg) were washed with water (10 x), bicarbonate buffer (pH 8, 10 x), and were suspended in bicarbonate buffer for 1 h. The labelled polypeptide was then added to the resin and kept overnight at room temperature. The resin was washed with bicarbonate buffer (pH 8) and water.

The fluorescence on the beads was recorded by using a fluorescence microscope and a digital camera. A mercury lamp equipped with an emission filter provided excitation in the visible blue range. The images of the beads were recorded in water.

The below formulas illustrate the building blocks which were used for the synthesis.

The below table lists the ligands and the corresponding apparent level of fluorescence determined by simply looking at the samples and comparing the apparent colour intensities between samples. On an arbitrary scale from 0 to 3, where 0 indicates no fluorescence and 3 indicates the strongest level of fluorescence observed.

| Number | composition | mark |
|---|---|---|
| L1#16 | D-Phe-(L)Arg-(L)Arg-Gly | 3 |
| LI#17 | L-Arg-D-Phe-(L)Arg-Gly | 3 |
| LI#19 | PPC-D-Pro-(L)Arg-Gly | 3 |
| L1#50 | PPC-D-Leu-PPC-Gly | 3 |
| LI#12 | INA-D-Phe-PPC-Gly | 2 |
| LI#14 | PPC-Aib-PPC-Gly | 2 |
| LI#16 | D-Phe-(L)Arg-(L)Arg-Gly | 2 |
| LI#17 | L-Arg-D-Phe-(L)Arg-Gly | 2 |
| LI#18 | L-Arg-(L)Arg-D-Phe-Gly | 2 |
| LI#20 | PPC-(L)Arg-D-Pro-Gly | 2 |

(continued)

| Number | composition | mark |
|---|---|---|
| L1#21 | D-Pro-PPC-(L)Arg-Gly | 2 |
| LI#24 | L-Arg-D-Pro-PPC-Gly | 2 |
| LI#26 | L-Arg-D-Lys-(L)Arg-Gly | 2 |
| LI#27 | L-Arg-(L)Arg-D-Lys-Gly | 2 |
| LI#41 | D-Lys-INA-(L)Arg-Gly | 2 |
| LI#44 | INA-(L)Arg-D-Lys-Gly | 2 |
| LI#53 | PPC-Ahx-PPC-Gly | 2 |
| LI#56 | PPC-Nle-PPC-Gly | 2 |
| LII#48 | SAA-(L)Arg-(L)Pro-Gly | 3 |
| LII#49 | SAA-(L)Pro-(L)Arg-Gly | 3 |
| LII#54 | DBHBA-(L)Arg-(L)Asn-Gly | 3 |
| LII#55 | DBHBA-(L)Asn-(L)Arg-Gly | 3 |
| LII#44 | 3HBA-(L)Arg-(L)Pro-Gly | 2 |
| LII#45 | 3HBA-(L)Pro-(L)Arg-Gly | 2 |
| LII#46 | 4HBA-(L)Arg-(L)Pro-Gly | 2 |
| LII#47 | 4HBA-(L)Pro-(L)Arg-Gly | 2 |

*Example 2. Chromatographic evaluation of ligands (from Example 1)*

<u>Preparinq the chromatography column</u>

The new resins were assessed in packed mode. 1 mL of each resin was packed into an HR5 chromatography column (GE Healthcare) applying standard conditions.

After packing, the column had a bed height of 3.9 cm and a total volume of 0.76 mL.

<u>Equilibration, Load, Elution</u>

Before loading the matrix with the monoclonal antibody, the column was equilibrated with 6 column volumes equilibration buffer containing 50 mM Na-phosphate (pH 7.0; cond. 9 mS $cm^{-1}$). After equilibration a monoclonal antibody solution in equilibration buffer (protein conc. 1.14 mg/mL) was loaded to the column. Subsequently, the column was washed with 6 column volumes of equilibration buffer and eluted with 6 column volumes of elution buffer 1 (25 mM sodium acetate pH 5.5; cond. 1.8 mS $cm^{-1}$) and 6 column volumes of elution buffer 2 (25 mM sodium acetate buffer pH 3.8; 0.5 mS $cm^{-1}$). The column was stored in 20% ethanol. Afterwards, the matrix was regenerated with 40 mM phosphoric acid and 20 mM sodium hydroxid. The flow rate for equilibration, load and wash was 60 cm/h, elution and regeneration was performed at a flow rate of 30 cm/h.

<u>Results for Ligand 55 coupled on Fractogel</u>

| Process step | Volume | mAb content | | |
|---|---|---|---|---|
| | [mL] | [mg/mL] | [mg] | Step yield [%] |
| Load | 6.6 | 1.10 | 7.26 | 100 |
| Flow trough | 6.5 | 0.19 | 1.24 | 17.0 |
| Wash | 4.5 | 0.02 | 0.09 | 1.20 |
| Elution 1 | 4.5 | 0.19 | 0.86 | 11.8 |
| Elution 2 | 4.0 | 1.05 | 4.2 | 57.9 |
| Regeneration | 1.5 | 0.49 | 0.74 | 10.1 |
| Recovery | | | | 98 |

Results for Ligand 16 coupled on Fractogel

| Process step | Volume | mAb content | | |
|---|---|---|---|---|
| | [mL] | [mg/mL] | [mg] | Step yield [%] |
| Load | 7.0 | 1.10 | 7.70 | 100 |
| Flow trough | 7.0 | 0.01 | 0.07 | 0.9 |
| Wash | 4.5 | 0.00 | 0.00 | 0.0 |
| Elution 1 | 5.0 | 1.13 | 5.65 | 73.4 |
| Elution 2 | 4.5 | 0.35 | 1.58 | 20.5 |
| Regeneration | 1.5 | 0.11 | 0.17 | 2.1 |
| Recovery | | | | 97 |

Summary and results:

Binding and elution of mAbs were shown with both ligands applying not optimized operation conditions. For ligand 55, only limited material is in the flow through whereas nothing was detected in the flow through with ligand 16.

*Example 3*

The ligands TMPPA-(L)Trp-(L)Arg-Gly-OH, DBHPA-(L)Arg-(L)Orn-Gly-OH, $(DPPA)_2$-DAP-(L)Arg-(L)Orn-Gly-OH and $(DBHBA)_2$-DAP-(L)Arg-(L)Arg-Gly-OH were synthesized on amino activated Toyopearl resin (supplied by Tosoh). The binding capacity of each of the resulting affinty resins (B2, B3, D1, and D2 respectively) towards mAb was evaluated by performing the folowing sequence of steps two times (cycle 1 and cycle 2),

1) passing an aqueous solution of mAb (50 mM Na-Phosphate, pH=7.0) through a column packed with the resin while recording the development of the UV absorbance of the permeate and collecting the liquid sample exiting the column (flow through),
2) passing an aqueous elution buffer (25 mM Acetate pH=3.7) through the column and collecting the liquid sample exiting the column (elution),
3) passing an aqueous sanitising buffer (1 M acetic acid) through the column and collecting the liquid sample exiting the column (regeneration/sanitation).

The mass of mAb, $m_i$, i=1,2,3, was then determined for each sample in each cycle.
The 'binding capacity' is calculated as the total mass of mAb added to the column, $m_0$, multiplied by the volume of buffer added to the column at the point where the UV absorbance has reached 5% of its terminal value, $V_{5\%}$, divided by the total volume of buffer used, $V_0$, and divided by the volume of wet resin, $V_{resin}$.

$$\text{Binding capacity} = \frac{V_{5\%}}{V_0} \times \frac{m_0}{V_{resin}}$$

The table below gives the results for B2, B3, D1, and D2 (Flow through = $m_1/m_0$, Elution = $m_2/m_0$, and Washing = $M_3/M_0$)

| **Resin** | **B2** | | **B3** | | **D1** | | **D2** | |
|---|---|---|---|---|---|---|---|---|
| Cycle | 1 | 2 | 1 | 2 | 1 | 2 | 1 | 2 |

(continued)

| Resin | | B2 | | B3 | | D1 | | D2 | |
|---|---|---|---|---|---|---|---|---|---|
| Binding capacity | (mg/mL) | 17 | 15 | 12 | 12 | 8 | 7 | 17 | 16 |
| Flow through | (%) | 20 | 22 | 19 | 20 | 31 | 32 | 10 | 12 |
| Elution | (%) | 23 | 26 | 47 | 47 | 60 | 63 | 47 | 49 |
| Regeneration | (%) | 43 | 48 | 33 | 30 | 8 | 5 | 26 | 27 |

It is clear from the table that all of the tested ligands have binding capacities higher than 5 mg/mL.

In order to determine the selectivity of resins B2 and B3 towards mAb, the experiment was repeated, but this time the crude fermentation supernatant was used in step 1. After the experiment, the purity of the samples was determined by SDS Page. The results are shown for B2, B3, D1, and D2 in figures 1, 2, 3, and 4, respectively.

It can be seen from Figures 1-4 that all four tested ligands show selectivity towards mAb with B2 and D1 being especially selective at the given conditions.

EMBODIMENTS

1. A solid support material having covalently immobilized thereon an affinity ligand, said ligand comprising one or more hydrophobic functional group(s) and one or more cationic functional group(s),
wherein at least one hydrophobic functional group is separated from at least one cationic functional group by a through bond distance of from 5 Å, to 20 Å,
wherein said ligand has a molecular weight of from 120 Da to 5,000 Da.

2. The solid support material according to embodiment 1, wherein said affinity resin has a binding capacity larger than 5 mg monoclonal antibody per mL of affinity resin.

3. The solid support material according to any one of the preceding embodiments, wherein the affinity ligand comprises one or more hydrophobic functional group(s) and one or more cationic functional group(s), wherein at least one hydrophobic functional group is separated from at least one cationic functional group by a through bond distance of from 5 Å, to 20 Å, and wherein said ligand has a molecular weight of from 120 Da to 1,500 Da.

4. The solid support material according to any one of the preceding embodiments, wherein the affinity ligand comprising or consisting of covalently linked residues $X_1$-$X_2$-$X_3$, wherein optionally $X_1$, $X_2$ and/or $X_3$ is associated with a linker residue.

5. The solid support material according to embodiment 4, wherein residue $X_1$ is selected from the group consisting of Arg, Phe, PPC, DBHBA, SAA, DAP, DAB, $(DBHBA)_2$-DAP, $(MDCA)_2$-DAP, DPBBA, DBBA, PCAA, DPPAA, Trp, TMPPA, and DBHPA.

6. The solid support material according to any one of the embodiments 4-5, wherein residue $X_2$ is selected from the group consisting of Arg, Asn, Leu, Lys, Phe, Pro, PPC, DAP, DAB, His, Trp, Tyr, and Ser.

7. The solid support material according to any one of the embodiments 4-6, wherein residue $X_3$ is selected from the group consisting of Arg, Asn, Pro, PPC, Asp, Orn, and (1H2NA)Dap.

8. The solid support material according to any one of the embodiments 4-7, wherein the ligand comprises or consists of 3 covalently linked residues, $X_1$-$X_2$-$X_3$,
wherein said covalently linked residues are further covalently linked to the linker L of the entity L-PM, wherein L is a linker, and PM is the solid support material, preferably a polymer matrix optionally in cross-linked and/or beaded form,
wherein $X_1$ is a natural or non-natural amino acid in D- and/or L-configuration, or a carboxylic acid residue comprising an optionally substituted aromatic group,
wherein $X_2$ is a natural or non-natural amino acid in either D- and/or L-configuration, or a carboxylic acid residue

comprising an optionally substituted aromatic group, with the proviso that $X_2$ is not a threonine residue, and wherein $X_3$ is a natural or non-natural amino acid in either D- and/or L-configuration, or a carboxylic acid residue comprising an optionally substituted aromatic group,
wherein at least one of $X_1$, $X_2$ and $X_3$ comprises a cationic functional group, and
wherein at least one of $X_1$, $X_2$ and $X_3$ comprises a hydrophobic functional group. wherein $X_1$ is selected from L-Arg, D-Lys, D-Phe, D-Pro, INA, PPC, DBHBA, 3HBA, 4HBA and SAA;
wherein $X_2$ is selected from L-Arg, L-Asn, D-Leu, D-Lys, D-Phe, D-Pro, L-Pro, AIB, AHX, INA, NLE and PPC; and
wherein $X_3$ is selected from L-Arg, L-Asn, D-Lys, D-Phe, D-Pro, L-Pro and PPC.

9. The solid support material according to any one of the preceding embodiments, wherein the ligand is one selected from the group consisting of
$H_2N$-(D)Phe-(L)Arg-(L)Arg-Gly-OH,
$H_2N$-(L)Arg-(D)Phe-(L)Arg-Gly-OH,
HN-PPC-(D)Pro-(L)Arg-Gly-OH,
HN-PPC-(D)Leu-PPC-Gly-OH,
DBBA-(L)His-(L)Arg-Gly-OH,
DBBA-His-Arg-Gly-OH,
DBBA-His-Arg-Arg-Gly-OH,
DPPBA-(L)Phe-(L)Arg-Gly-OH,
PCAA-(L)Phe-(L)Arg-Gly-OH,
DPPBA-(L)Lys-(L)Arg-Gly-OH,
SAA-(L)Arg-(L)Pro-Gly-OH,
SAA-(L)Pro-(L)Arg-Gly-OH,
DBHBA-(L)Arg-(L)Asn-Gly-OH,
DBHBA-(L)Asn-(L)Arg-Gly-OH,
DPPBA-(L)Trp-(L)Arg-Gly-OH,
$(MDCA)_2$-DAP-(L)Arg-(L)Orn-Gly-OH,
$(MDCA)_2$-DAP-(L)Arg-(L)Asp-Gly-OH,
DPPAA-(L)Phe-(L)Arg-Gly-OH,
DPPAA-PPC-(L)Arg-Gly-OH,
DBHBA-(D)Phe-(D)Arg-Gly-OH,
DPPAA-(D)tyr-(D)Arg-Gly-OH,
$H_2N$-(D)Trp-(D)Ser-(1H2NA)DAP-Aib-OH,
$H_2N$-(L)His-(D)Ser-(1H2NA)DAP-Aib-OH,
$(DPPA)_2$-DAP-(L)Arg-(L)Orn-Gly-OH,
$(DBHBA)_2$-DAP-(L)Arg-(L)Arg-Gly-OH,
DBHBA-DAP-Arg-Gly-OH,
DBHBA-DAP-Arg-Arg-Gly-OH,
$(DBHBA)_2$-DAP-Arg-Gly-OH,
$(DBHBA)_3$-DAP-Arg-Arg-Gly-OH,
$(DBHBA)_3$-DAP-Arg-Arg-Gly-OH,
TMPPA-(L)Trp-(L)Arg-Gly-OH, and
DBHPA-(L)Arg-(L)Orn-Gly-OH.

10. The solid support material according to any one of the preceding embodiments, wherein the ligand is selected from the group consisting of DBBA-(L)His-(L)Arg-Gly-OH and $(DBHBA)_2$-DAP-(L)Arg-(L)Arg-Gly-OH.

11. A solid support material having covalently immobilized thereon an affinity ligand, said ligand having one or more hydrophobic functional group(s) and one or more heteroaromatic functional group(s),
wherein at least one hydrophobic functional group is separated from at least one heteroaromatic functional group by a through bond distance of from 5 Å, to 20 Å, and
wherein said ligand has a molecular weight of from 120 Da to 5,000 Da.

12. The solid support material according to embodiment 11, wherein said affinity resin has a binding capacity larger than 5 mg monoclonal antibody per mL of affinity resin.

13. The solid support material according to any one of embodiments 11-12, wherein the ligand is $(TEBA)_2$-DAP-(L)Trp-(L)Trp-Gly-OH.

14. A method for the isolation of antibodies or derivatives thereof, the method comprising the steps of (i) providing a solid support material having covalently immobilized thereon an affinity ligand as defined in any one of embodiments 1-13, (ii) providing a sample containing an antibody having an affinity for said ligand, (iii) contacting said ligand with said sample containing said antibody, (iv) binding selectively said antibody when said antibody is contained in said sample and (v) isolating selectively said antibody when said antibody is contained in said sample.

## Claims

1. A solid support material having covalently immobilized thereon an affinity ligand, said ligand comprising one or more hydrophobic functional group(s) and one or more cationic functional group(s),
wherein at least one hydrophobic functional group is separated from at least one cationic functional group by a through bond distance of from 5 Å to 20 Å,
wherein said ligand consists of less than 5 residues and has a molecular weight of from 120 Da to 1,000 Da.

2. The solid support material according to claim 1, wherein said affinity resin has a binding capacity larger than 5 mg monoclonal antibody per mL of affinity resin.

3. The solid support material according to any one of the preceding claims, wherein the affinity ligand comprising or consisting of covalently linked residues $X_1$-$X_2$-$X_3$, wherein optionally $X_1$, $X_2$ and/or $X_3$ is associated with a linker residue.

4. The solid support material according to claim 3, wherein residue $X_1$ is selected from the group consisting of Arg, Phe, PPC, DBHBA, SAA, DAP, DAB, $(DBHBA)_2$-DAP, $(MDCA)_2$-DAP, DPBBA, DBBA, PCAA, DPPAA, Trp, TMPPA, and DBHPA.

5. The solid support material according to any one of the claims 3-4, wherein residue $X_2$ is selected from the group consisting of Arg, Asn, Leu, Lys, Phe, Pro, PPC, DAP, DAB, His, Trp, Tyr, and Ser.

6. The solid support material according to any one of the claims 3-5, wherein residue $X_3$ is selected from the group consisting of Arg, Asn, Pro, PPC, Asp, Orn, and (1H2NA)Dap.

7. The solid support material according to any one of the claims 3-6, wherein the ligand comprises or consists of 3 covalently linked residues, $X_1$-$X_2$-$X_3$,
wherein said covalently linked residues are further covalently linked to the linker L of the entity L-PM, wherein L is a linker, and PM is the solid support material, preferably a polymer matrix optionally in cross-linked and/or beaded form,

wherein $X_1$ is a natural or non-natural amino acid in D- and/or L-configuration, or a carboxylic acid residue comprising an optionally substituted aromatic group,
wherein $X_2$ is a natural or non-natural amino acid in either D- and/or L-configuration, or a carboxylic acid residue comprising an optionally substituted aromatic group, with the proviso that $X_2$ is not a threonine residue, and
wherein $X_3$ is a natural or non-natural amino acid in either D- and/or L-configuration, or a carboxylic acid residue comprising an optionally substituted aromatic group,
wherein at least one of $X_1$, $X_2$ and $X_3$ comprises a cationic functional group, and
wherein at least one of $X_1$, $X_2$ and $X_3$ comprises a hydrophobic functional group.
wherein $X_1$ is selected from L-Arg, D-Lys, D-Phe, D-Pro, INA, PPC, DBHBA, 3HBA, 4HBA and SAA;
wherein $X_2$ is selected from L-Arg, L-Asn, D-Leu, D-Lys, D-Phe, D-Pro, L-Pro, AIB, AHX, INA, NLE and PPC; and
wherein $X_3$ is selected from L-Arg, L-Asn, D-Lys, D-Phe, D-Pro, L-Pro and PPC.

**8.** The solid support material according to any one of the preceding claims, wherein the ligand is one selected from the group consisting of
$H_2N$-(D)Phe-(L)Arg-(L)Arg-Gly-OH,
$H_2N$-(L)Arg-(D)Phe-(L)Arg-Gly-OH,
HN-PPC-(D)Pro-(L)Arg-Gly-OH,
DBBA-(L)His-(L)Arg-Gly-OH,
DBBA-His-Arg-Gly-OH,
DBBA-His-Arg-Arg-Gly-OH,
DPPBA-(L)Phe-(L)Arg-Gly-OH,
PCAA-(L)Phe-(L)Arg-Gly-OH,
DPPBA-(L)Lys-(L)Arg-Gly-OH,
SAA-(L)Arg-(L)Pro-Gly-OH,
SAA-(L)Pro-(L)Arg-Gly-OH,
DBHBA-(L)Arg-(L)Asn-Gly-OH,
DBHBA-(L)Asn-(L)Arg-Gly-OH,
DPPBA-(L)Trp-(L)Arg-Gly-OH,
$(MDCA)_2$-DAP-(L)Arg-(L)Orn-Gly-OH,
$(MDCA)_2$-DAP-(L)Arg-(L)Asp-Gly-OH,
DPPAA-(L)Phe-(L)Arg-Gly-OH,
DPPAA-PPC-(L)Arg-Gly-OH,
DBHBA-(D)Phe-(D)Arg-Gly-OH,
DPPAA-(D)tyr-(D)Arg-Gly-OH,
$(DPPA)_2$-DAP-(L)Arg-(L)Orn-Gly-OH,
$(DBHBA)_2$-DAP-(L)Arg-(L)Arg-Gly-OH,
DBHBA-DAP-Arg-Gly-OH,
DBHBA-DAP-Arg-Arg-Gly-OH,
$(DBHBA)_2$-DAP-Arg-Gly-OH,
$(DBHBA)_3$-DAP-Arg-Arg-Gly-OH,
TMPPA-(L)Trp-(L)Arg-Gly-OH, and
DBHPA-(L)Arg-(L)Orn-Gly-OH.

**9.** The solid support material according to any one of the preceding claims, wherein the ligand is selected from the group consisting of DBBA-(L)His-(L)Arg-Gly-OH and $(DBHBA)_2$-DAP-(L)Arg-(L)Arg-Gly-OH.

**10.** A method for the isolation of antibodies or derivatives thereof, the method comprising the steps of (i) providing a solid support material having covalently immobilized thereon an affinity ligand as defined in any one of claims 1-9, (ii) providing a sample containing an antibody having an affinity for said ligand, (iii) contacting said ligand with said sample containing said antibody, (iv) binding selectively said antibody when said antibody is contained in said sample and (v) isolating selectively said antibody when said antibody is contained in said sample.

FIGURE 1

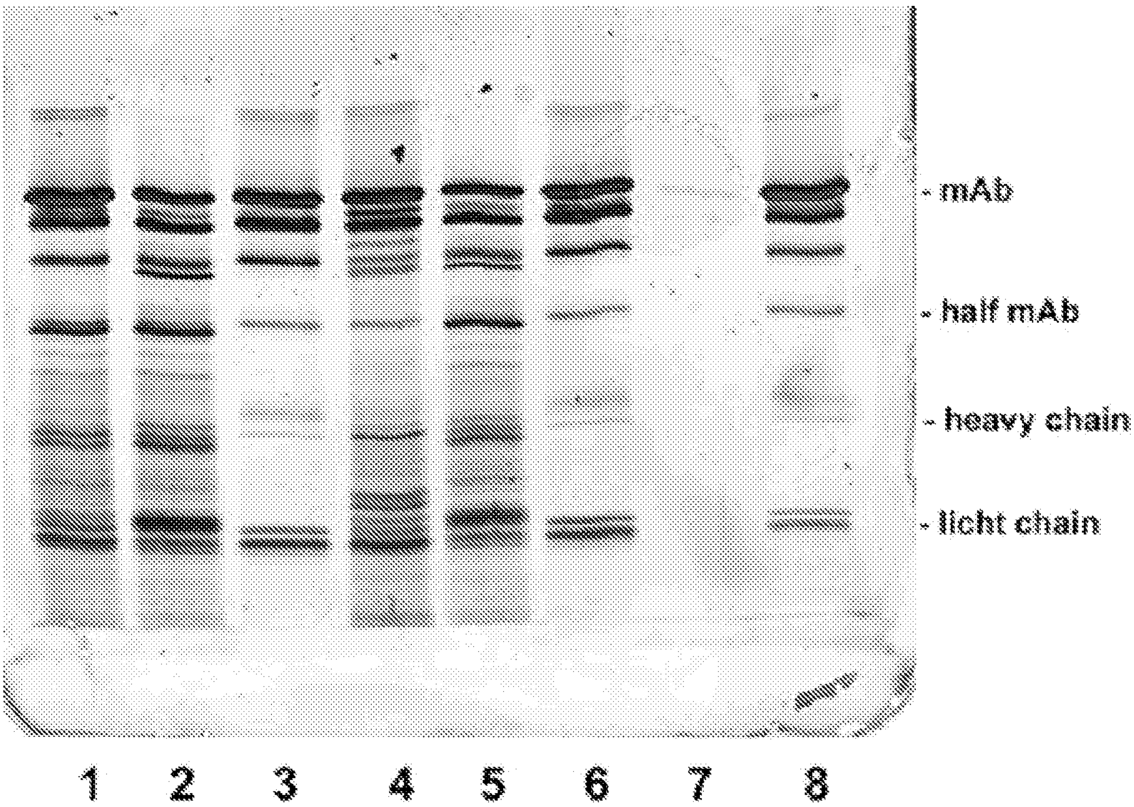


FIGURE 2

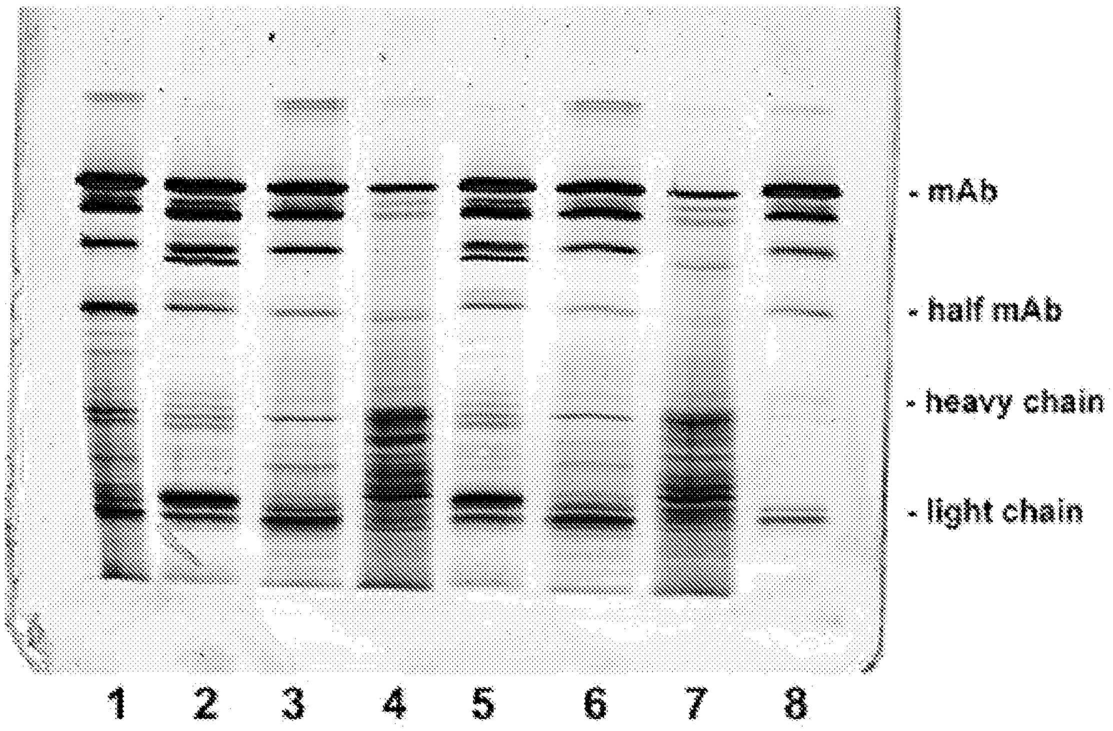

FIGURE 3

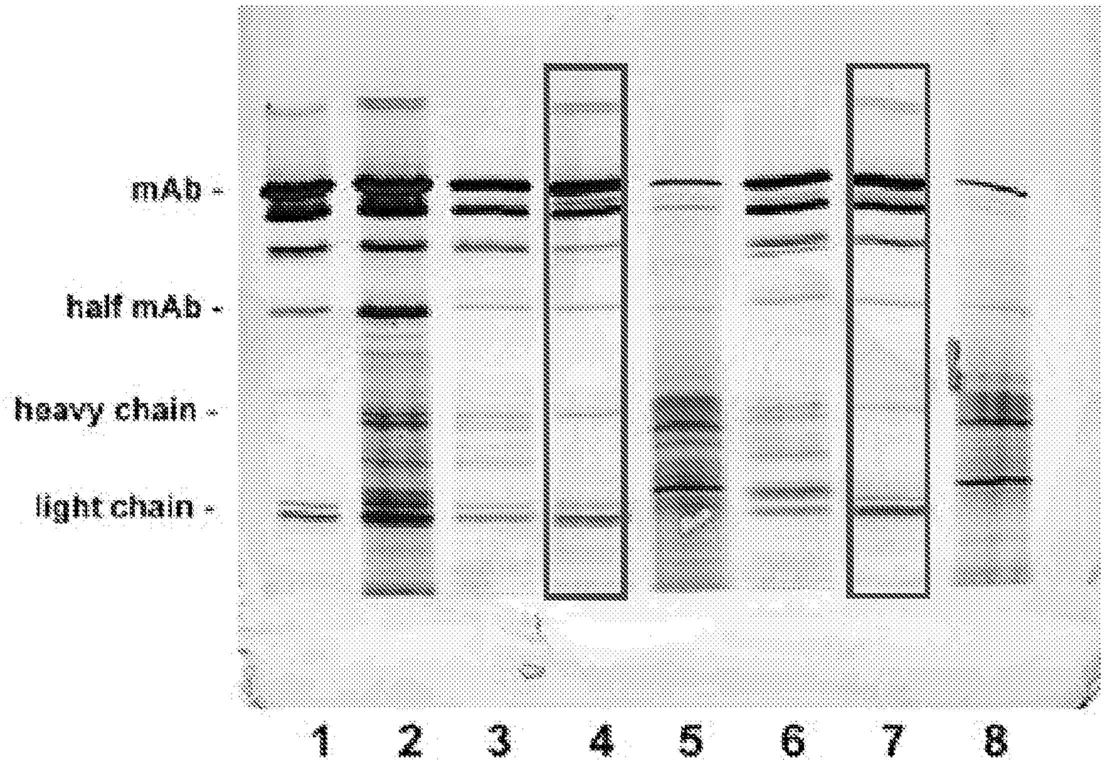


FIGURE 4

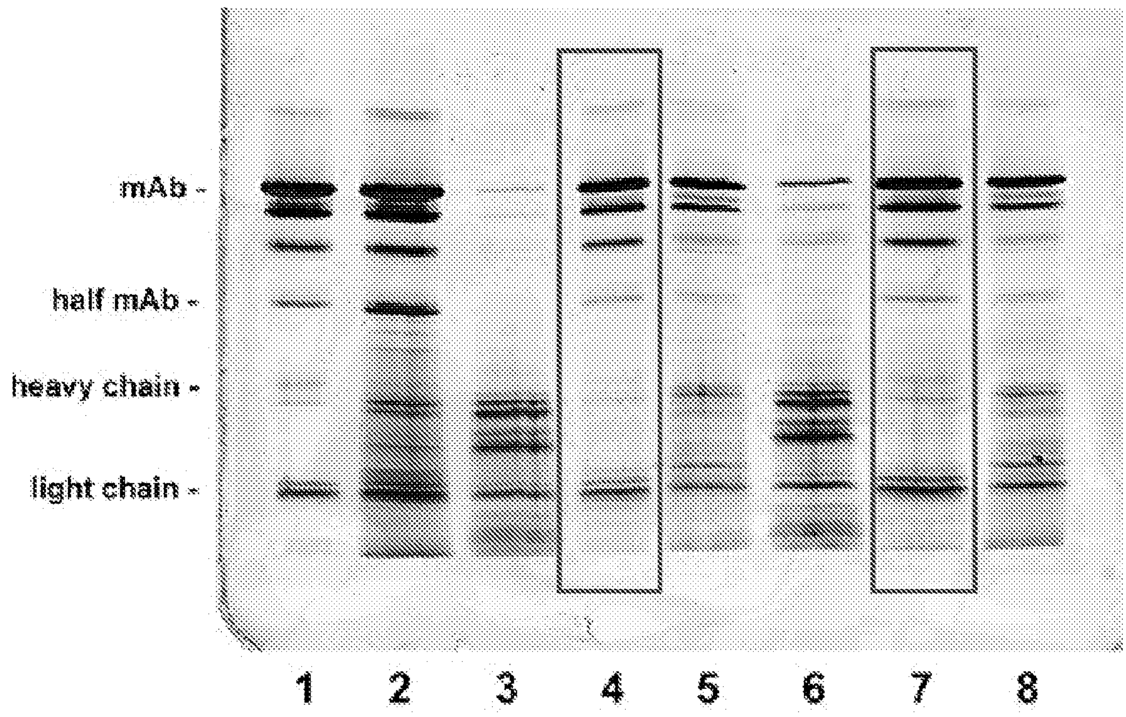

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Lehninger.** Biochemistry. Worth Publishers, 1975, 71-92
- **Roberts ; Vellaccio.** The Peptides: Analysis, Synthesis, Biology. Academic Press, Inc, 1983, vol. 5, 341
- **Adams et al.** *J.Org.Chem.,* 1998, vol. 63, 3706-3716
- **Grøtli et al.** *J.Combi.Chem.,* 2000, vol. 2, 108-119
- **Hermanson ; Krishna ; Mallia ; Smith.** Immobilized Affinity Ligand Techniques. Academic Press, 1992

SEQUENCE LISTING

<110> Versamatrix A/S

<120> Antibody binding affinity ligands

<130> 16115PCT00

<160> 23

<170> PatentIn version 3.3

<210> 1
<211> 4
<212> PRT
<213> Artificial

<220>
<223> artificial

<400> 1

Phe Arg Arg Gly
1

<210> 2
<211> 4
<212> PRT
<213> Artificial

<220>
<223> artificial

<400> 2

Arg Phe Arg Gly
1

<210> 3
<211> 4
<212> PRT
<213> Artificial

<220>
<223> artificial

<220>
<221> MOD_RES
<222> (1)..(1)

<400> 3

Xaa Pro Arg Gly
1

<210> 4
<211> 4
<212> PRT
<213> Artificial

<220>
<223> artificial

<220>
<221> MOD_RES
<222> (1)..(1)

<220>
<221> MOD_RES
<222> (3)..(3)

<400> 4

Xaa Leu Xaa Gly
1

<210> 5
<211> 4
<212> PRT
<213> Artificial

<220>
<223> artificial

<400> 5

His Arg Arg Gly
1

<210> 6
<211> 4
<212> PRT
<213> Artificial

<220>
<223> artificial

<220>
<221> MOD_RES
<222> (1)..(1)

<220>
<221> MOD_RES
<222> (3)..(3)

<400> 6

Xaa Arg Xaa Gly
1

<210> 7
<211> 4
<212> PRT
<213> Artificial

<220>
<223> Artificial

<220>
<221> MOD_RES
<222> (1)..(1)

<400> 7

Xaa Arg Asp Gly
1

<210> 8
<211> 4
<212> PRT
<213> Artificial

<220>
<223> Artificial

<220>
<221> MOD_RES
<222> (3)..(4)

<400> 8

Trp Ser Xaa Xaa
1

<210> 9
<211> 4
<212> PRT
<213> Artificial

<220>
<223> Artificial

<220>
<221> MOD_RES
<222> (3)..(4)

<400> 9

His Ser Xaa Xaa
1

<210> 10
<211> 4
<212> PRT
<213> Artificial

<220>
<223> Artificial

<220>
<221> MOD_RES
<222> (1)..(1)

<220>
<221> MOD_RES
<222> (3)..(3)

<400> 10

Xaa Arg Xaa Gly
1

<210> 11
<211> 4
<212> PRT
<213> Artificial

<220>
<223> Artificial

<220>
<221> MOD_RES
<222> (1)..(1)

<400> 11

Xaa Arg Arg Gly
1

<210> 12
<211> 4
<212> PRT
<213> Artificial

<220>
<223> Artificial

<220>
<221> MOD_RES
<222> (1)..(1)

<400> 12

Xaa Trp Trp Gly
1

<210> 13
<211> 4
<212> PRT
<213> Artificial

<220>
<223> Artificial

<220>
<221> MOD_RES
<222> (1)..(1)

<220>
<221> MOD_RES
<222> (3)..(3)

<400> 13

Xaa Phe Xaa Gly
1

<210> 14
<211> 4
<212> PRT
<213> Artificial

<220>
<223> Artificial

<220>
<221> MOD_RES
<222> (1)..(3)

<400> 14

Xaa Xaa Xaa Gly
1

<210> 15
<211> 4
<212> PRT
<213> Artificial

<220>
<223> Artificial

<400> 15

Arg Arg Phe Gly
1

<210> 16
<211> 4
<212> PRT
<213> Artificial

<220>
<223> Artificial

<220>
<221> MOD_RES
<222> (1)..(1)

<400> 16

Xaa Arg Pro Gly
1

<210> 17
<211> 4
<212> PRT
<213> Artificial

<220>
<223> Artificial

<220>
<221> MOD_RES
<222> (2)..(2)

<400> 17

Pro Xaa Arg Gly
1

<210> 18
<211> 4
<212> PRT
<213> Artificial

<220>
<223> Artificial

<220>
<221> MOD_RES
<222> (3)..(3)

<400> 18

Arg Pro Xaa Gly
1

<210> 19
<211> 4
<212> PRT
<213> Artificial

<220>
<223> Artificial

<400> 19

Arg Lys Arg Gly
1

<210> 20
<211> 4
<212> PRT
<213> Artificial

<220>
<223> Artificial

<400> 20

Arg Arg Lys Gly
1

<210> 21
<211> 4
<212> PRT
<213> Artificial

<220>
<223> Artificial

<220>
<221> MOD_RES
<222> (2)..(2)

<400> 21

Lys Xaa Arg Gly
1

<210> 22
<211> 4
<212> PRT
<213> Artificial

<220>
<223> Artificial

<220>
<221> MOD_RES

<222> (1)..(1)

<400> 22

Xaa Arg Lys Gly
1

<210> 23
<211> 4
<212> PRT
<213> Artificial

<220>
<223> Artificial

<220>
<221> MOD_RES
<222> (1)..(3)

<400> 23

Xaa Xaa Xaa Gly
1